# EUROPÄISCHE PATENTANMELDUNG

(11) **EP 4 299 656 A1**
(43) Veröffentlichungstag der Anmeldung: **03.01.2024**
(21) Anmeldenummer: 22182455.0
(22) Anmeldetag: 01.07.2022
(51) Int. Cl.: C08J 9/14, C08G 18/18, C08G 18/48, C08G 18/76, C08G 18/09, C08G 18/20, C08G 18/38, C08G 18/66, C08G 18/50, C07C 233/64

(54) **HERSTELLUNG VON PROPOXYLIERTEN BENZOLDICARBONSÄUREAMIDEN UND DEM ENTSPRECHENDEN POLYURETHANSCHAUM**

(71) Anmelder: Evonik Operations GmbH, 45128 Essen (DE)
(72) Erfinder: GLOS, Martin, 46325 Borken (DE); SCHUBERT, Frank, 47506 Neukirchen-Vluyn (DE)
(74) Vertreter: Evonik Patent Association

(57) **Zusammenfassung**

Beschrieben werden
(a) Verbindungen der Formel (I), 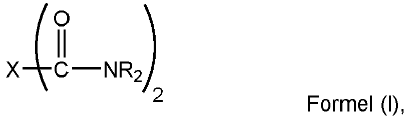 wobei
X
ein Phenylenrest ist,
R
jeweils unabhängig voneinander ausgewählt ist aus Resten der Formel (II), -[A-O]ₙ-H Formel (II),
A
ein Propylenrest ist, und
n
eine ganze Zahl ≥0 ist,
mit der Maßgabe, dass die Verbindung der Formel (I) mindestens 2 Einheiten A aufweist;
(b) Mischungen umfassend oder bestehend aus Verbindungen (a);
(c) Verfahren zur Herstellung von Verbindungen (a);
(d) Zusammensetzungen zur Herstellung von Polyurethanschaum, die Verbindungen (a) enthalten;
(e) Verfahren zur Herstellung von Polyurethanschaum unter Einsatz einer Zusammensetzung (d);
(f) Polyurethanschaumstoff, erhältlich durch das Verfahren (e);
(g) Verwendung des Polyurethanschaumstoffs (f) als Dämmplatten und/oder Isolationsmittel, vorzugsweise für Kühlapparaturen;
(h) Verwendung einer Verbindung (a) zur Verbesserung der Isoliereigenschaften von Polyurethanschaumstoffen.

## Beschreibung

Die vorliegende Erfindung liegt auf dem Gebiet der Polyurethanschäume. Insbesondere betrifft sie die Herstellung von Polyurethanschäumen unter Verwendung spezieller Polyole, sowie weiterhin die Verwendung der Schäume, die damit hergestellt wurden.

Unter Polyurethan (PU) wird im Rahmen der vorliegenden Erfindung insbesondere ein Produkt erhältlich durch Reaktion von Polyisocyanaten und Polyolen bzw. Verbindungen mit Isocyanat-reaktiven Gruppen verstanden. Es können hierbei neben dem Polyurethan auch weitere funktionelle Gruppen gebildet werden, wie z.B. Uretdione, Carbodiimide, Isocyanurate, Allophanate, Biurete, Harnstoffe und/oder Uretimine. Daherwerden unter PU im Sinne der vorliegenden Erfindung sowohl Polyurethan als auch Polyisocyanurat, Polyharnstoffe und Uretdion-, Carbodiimid-, Allophanat-, Biuret- und Uretimin-Gruppen enthaltende Polyisocyanat-Reaktionsprodukte verstanden. Unter Polyurethanschaum (PU-Schaum) wird im Rahmen der vorliegenden Erfindung insbesondere Schaum verstanden, der als Reaktionsprodukt basierend auf Polyisocyanaten und Polyolen bzw. Verbindungen mit Isocyanat-reaktiven Gruppen erhalten wird. Es können hierbei neben dem Namen gebenden Polyurethan auch weitere funktionelle Gruppen gebildet werden, wie z.B. Allophanate, Biurete, Harnstoffe, Carbodiimide, Uretdione, Isocyanurate oder Uretimine.

Polyurethanschaum wird üblicherweise auch als Polyurethanschaumstoff bezeichnet. Die Begriffe "Schaum" und "Schaumstoff" werden hier daher auch synonym verwendet.

Bei der Herstellung von Polyurethan- und Polyisocyanurat-Schaumstoffen werden unterschiedliche Polyole eingesetzt, welche die Gebrauchseigenschaften und den Herstellprozess des Schaums in wesentlichem Maße beeinflussen. Hierbei sind vor allem das thermische Isolationsvermögen und die mechanischen Eigenschaften des Schaumstoffes wichtig.

Polyole, die durch Alkoxylierung von aromatischen Aminen wie Anilin, Toluidin, Toluoldiaminen oder Diaminobenzolen hergestellt werden, sind dem Fachmann lange bekannt. Als Beispiele seien folgende Schriften genannt: US 7691913, US 4391728, US 4562290 und DE 3740634.

EP 1806374 beschreibt Polyurethan-Hartschaumstoffe für Kühlgeräte mit verbesserter Haftung durch Verwendung von alkoxylierten Monoaminen wie z.B. Anilin. Diese Verbindungen können jedoch die Verarbeitungseigenschaften der Schaumstoffe negativ beeinflussen.

WO 0063276 offenbart ein Polyolgemisch für die Herstellung von Polyurethan-Hartschäumen. Dieses Gemisch enthält Additionsprodukte von Alkylenoxiden an aromatische Di- oder Polyamine. Als gegenüber Isocyanatgruppen reaktiven Verbindungen kann auch ein Polyesterpolyol vorliegen.

Bekannt sind auch aromatische Polyole, die sich von aromatischen Carbonsäuren, deren Estern oder Anhydriden ableiten.

EP 1203763 beschreibt N,N'-Bis(hydroxyalkyl)benzamide, die durch Umsetzung von Benzoesäurederivaten wie Benzoylchlorid mit Alkanolaminen wie Diisopropanolamin in einem Lösemittel hergestellt werden.

EP 0068281 offenbart aromatische Amide als Zellöffner für Polyurethanschäume, die durch Umsetzung von z.B. Terephthalsäuredimethylester oder Naphthalintetracarbonsäuretetramethylester mit Diethanolamin hergestellt werden. Die Reaktionsprodukte sind bei Raumtemperatur kristalline oder glasartig erstarrte Feststoffe und werden zur Herstellung offenzelliger, elastischer Polyurethanschäume verwendet.

JP 2000072732 beschreibt Amidgruppen enthaltende Polyole für Polyurethane, die durch eine Umsetzung von Dicarbonsäurediestern mit Aminoalkoholen synthetisiert werden. Zu den genannten Produkten gehören N,N'-Bis(2-hydroxyethyl)terephthalamide, N,N'-Bis(2-hydroxyethyl)phthalamide und N,N'-Bis(2-hydroxyethyl)isophthalamide.

JP 04013757 und JP 03199253 beschreiben hitzebeständige Polyester auf Basis N,N'-bis(2-hydroxyethyl)isophthalamide.

JP 07329420 offenbart Alkanolamide der Hydroxybenzoesäure, wie z.B. 2-Hydroxy-N-(2-hydroxypropyl)-benzamid, und deren Verwendung zur Herstellung von Speichermedien.

CN 112142612 beschreibt ebenfalls die Herstellung von Hydroxyalkylamiden. So wird ein Dialkyldicarbonsäureester wie Dimethylphthalat mit dem Überschuss eines Dialkylalkanolamins wie Diisopropanolamin zur Reaktion gebracht. Das Rohprodukt wird anschließend durch ein Extraktionsverfahren und abschließende Destillation gereinigt.

CN 111960963 beschreibt eine alternative Route durch Reaktion von Phthalsäureanhydrid mit Diisopropanolamin in Gegenwart von Toluol als Schleppmittel in der Wasserdestillation. Das fertige Polyol wird zur Herstellung von Polyurethanschäumen mit verbesserter Festigkeit und chemischer Beständigkeit verwendet.

CN 111909117 offenbart nach ähnlichem Verfahren hergestellte Tetrakis(2-hydroxypropyl)phthalamide, die in einem nachfolgenden Schritt mit Epichlorhydrin zu Epoxidharzen umgesetzt werden.

US 2841580 und US 2824018 beschreiben Terephthalamide aus Terephthalsäurederivaten und Diethanolamin, die für die Celluloseproduktion eingesetzt werden.

DE 2350780 offenbart Polyamide aus Phthalsäureanhydrid und Monoethanolamin, die anschließend ethoxyliert und propoxyliert werden, als Additive für bedruckbare Textilifasern.

M. lonescu et al., Proceedings of the APC Conference, Polyurethanes Expo 2001, Columbus OH, USA, 2001, S. 607-608 offenbart propoxylierte Reaktionsprodukte aus Benzoldicarbonsäuredimethylestern und Diethanolamin, die in Polyurethanschäumen die mechanischen Eigenschaften verbessern.

M. lonescu, "Polyol Special Polyol Structures For Rigid Polyurethane Foams", Polyols for Polyurethanes, Volume 2, S. 277-288 offenbart ebenfalls propoxylierte Reaktionsprodukte aus Benzoldicarbonsäuredimethylestern und Diethanolamin, die in Polyurethanschäumen die mechanischen Eigenschaften verbessern.

US 444223 beschreibt ein Verfahren, bei dem Polyethylenterephthalat mit Diethanolamin aminolytisch gespalten und das erhaltene Amid nach vorheriger Aufbereitung propoxyliert wird. Die amidfunktionellen Polyole dienen zur Fertigung von Polyurethanhartschäumen mit verbesserter Flammwidrigkeit.

Keines der vorgenannten Dokumente offenbart propoxylierte Benzoldicarbonsäureamide, die frei von Oxyethylen-Gruppen sind.

Der Herstellung und den Einsatzmöglichkeiten aromatischer amidfunktioneller Polyole sind derzeit häufig Grenzen gesetzt, z.B. durch deren Kristallisationsneigung und hohen Schmelz-/Erweichungspunkte, die eine betriebliche Handhabung schwierig bis unmöglich machen. Die Amidierung erfordert häufig den Einsatz von Lösemitteln, Schleppmitteln in der Wasserdestillation oder es Bedarf der Aufreinigung z.B. mittels Extraktion. Die Alkoxylierung der meist festen aromatischen Amide stellt nach Stand der Technik eine große technische Herausforderung dar.

Es bestand weiterhin der Bedarf an aromatischen amidfunktionellen Polyolen, die neben den bekannten Vorteilen aromatischen amidfunktionellen Polyolen, bei der Herstellung von PU-Schäumen zu einer verbesserten Aushärtung des Reaktionsgemisches (geringere Nachexpansion) und zu besseren Isolationseigenschaften führen. Zudem bestand der Wunsch nach Polyol-Systemen, die eine verbesserte Lagerstabilität zeigen und eine geringere Neigung zur eine Phasentrennung im Sinne eines "Aufrahmens" zeigen.

Aufgabe der vorliegenden Erfindung war es daher, zumindest einen Nachteil des Standes der Technik zu überwinden. Es bestand insbesondere die Aufgabe, Polyole bereitzustellen, die bei der Herstellung von Polyurethanschäumen zu besonders vorteilhafte Eigenschaften führen, wie eine verbesserte Aushärtung des Reaktionsgemisches (geringere Nachexpansion), verbesserte Isolationseigenschaften, sowie zu einer verbesserten Lagerstabilität des Polyol-Systems, sodass die Neigung zur Phasentrennung im Sinne eines "Aufrahmens" verringert oder sogar überhaupt nicht vorhanden ist.

Überraschenderweise wurde nun gefunden, dass spezielle propoxylierte Benzoldicarbonsäureamide, wie in den Ansprüchen beschrieben, diese Aufgabe lösen. 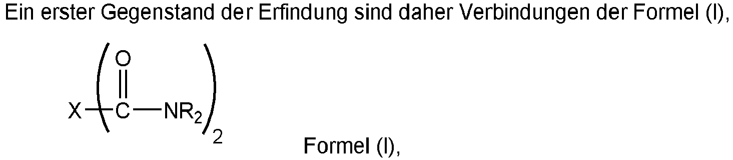 wobei
- X: ein Phenylenrest ist,
- R: jeweils unabhängig voneinander ausgewählt ist aus Resten der Formel (II), -[A-O]ₙ-H Formel (II),
- A: ein Propylenrest ist, und
- n: eine ganze Zahl ≥0 ist,
mit der Maßgabe, dass die Verbindung der Formel (I) mindestens 2 Einheiten A aufweist.

Ein weiterer Gegenstand der Erfindung ist eine Mischung umfassend oder bestehend aus den erfindungsgemäßen Verbindungen der Formel (I), dadurch gekennzeichnet, dass die mittlere Anzahl der Einheiten A pro Molekül der Verbindung der Formel (I) von 2 bis 14, vorzugsweise von 4 bis 12, insbesondere von 6 bis 10 beträgt.

Noch ein weiterer Gegenstand der Erfindung ist ein Verfahren zur Herstellung von einer oder mehreren erfindungsgemäßen Verbindungen der Formel (I) umfassend die Schritte:
a) Umsetzung mindestens einer aromatischen Verbindung ausgewählt aus der Gruppe bestehend aus Benzoldicarbonsäure, Benzoldicarbonsäureester und Benzoldicarbonsäureanhydrid mit mindestens einem Aminoalkohol ausgewählt aus der Gruppe bestehend aus Isopropanolamin und Diisopropanolamin, optional in Gegenwart eines alkalischen Katalysators, zu mindestens einem aromatischen Amid;
b) Umsetzung des mindestens einen aromatischen Amids mit Propylenoxid zu mindestens einer Verbindung der Formel (I).

Noch ein weiterer Gegenstand der Erfindung ist eine Zusammensetzung zur Herstellung von Polyurethanschaum, umfassend mindestens eine Polyisocyanat-Komponente, mindestens eine Polyol-Komponente, optional mindestens einen Katalysator, der die Ausbildung einer Urethan- oder Isocyanurat-Bindung katalysiert, und optional mindestens ein Treibmittel, dadurch gekennzeichnet, dass mindestens eine Polyol-Komponente ausgewählt ist aus den erfindungsgemäßen Verbindungen der Formel (I).

Noch ein weiterer Gegenstand der Erfindung ist ein Verfahren zur Herstellung von Polyurethanschaum, durch Umsetzung mindestens einer Polyol-Komponente mit mindestens einer Polyisocyanat-Komponente unter Einsatz der erfindungsgemäßen Zusammensetzung.

Noch ein weiterer Gegenstand der Erfindung ist ein Polyurethanschaumstoff, erhältlich durch das erfindungsgemäße Verfahren zur Herstellung von Polyurethanschaum.

Noch ein weiterer Gegenstand ist die Verwendung des erfindungsgemäßen Polyurethanschaumstoffs als Dämmplatten und/oder Isolationsmittel, vorzugsweise für Kühlapparaturen.

Noch ein weiterer Gegenstand der Erfindung ist die Verwendung der erfindungsgemäßen Verbindung der Formel (I) oder der erfindungsgemäßen Mischung zur Verbesserung der Isoliereigenschaften von Polyurethanschaumstoffen, insbesondere unter Einsatz der erfindungsgemäßen Zusammensetzung.

Vorteilhafte Ausgestaltungen der Gegenstände der Erfindung sind den Ansprüchen, den Beispielen und der Beschreibung zu entnehmen. Darüber hinaus wird ausdrücklich darauf hingewiesen, dass die Offenbarung zu den Gegenständen der vorliegenden Erfindung alle Kombinationen von einzelnen Merkmalen der vorliegenden bzw. nachfolgenden Beschreibung der Erfindung und der Patentansprüche einschließt. Insbesondere gelten Ausführungsformen eines erfindungsgemäßen Gegenstands mutatis mutandis auch für die Ausführungsformen der anderen erfindungsgemäßen Gegenstände.

Wie oben bereits erläutert handelt es sich bei den speziellen propoxylierten Benzoldicarbonsäureamiden um Verbindungen der Formel (I), 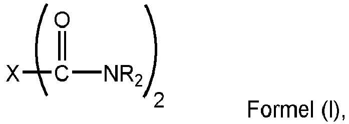 wobei
- X: ein Phenylenrest ist,
- R: jeweils unabhängig voneinander ausgewählt ist aus Resten der Formel (II), -[A-O]ₙ-H Formel (II),
- A: ein Propylenrest ist, und
- n: eine ganze Zahl ≥0 ist,
mit der Maßgabe, dass die Verbindung der Formel (I) mindestens 2 Einheiten A aufweist.

Der Phenylenrest X ist ein aromatischer Rest ausgewählt aus der Gruppe der folgende zweibindigen aromatischen Reste:

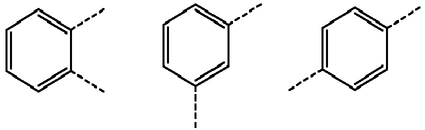

Diese Reste werden (von links nach rechts) als ortho-Phenylenrest, meta-Phenylenrest bzw. para-Phenylenrest bezeichnet. Die kovalenten Bindungen des Phenylenrests, über die der Phenylenrest an die beiden in Formel (I) dargestellten Amidgruppen gebunden ist, sind gestrichelt dargestellt. Besonders bevorzugt ist X ein ortho-Phenylenrest.

Die Verbindung der Formel (I) weist zweibindige Gruppen A auf, wobei A für einen Propylenrest steht. Ein Propylenrest ist ein zweibindiger Kohlenwasserstoffrest der Formel -(C₃H₆)- auf. Der Propylenrest liegt vorzugsweise jeweils unabhängig voneinander in den räumlichen Orientierungen -(CH₂-CH(CH₃))- oder -(CH(CH₃)-CH₂)- im Rest der Formel (II) vor, insbesondere in der räumlichen Orientierung -(CH₂-CH(CH₃))-, wobei jeweils die in Formel (II) gewählte räumliche Orientierung zu Grunde zu legen ist, also eine räumliche Orientierung bei der der Rest der Formel (II) -[A-O]ₙ-H links an N gebunden vorliegt und rechts eine Hydroxygruppe aufweist. Der Propylenrest kann auch als Kohlenwasserstoffrest der Formel -(CH₂-CH₂-CH₂)- vorliegen. Dies ist allerdings weniger bevorzugt. Die Verbindung der Formel (I) weist mindestens 2 Einheiten A pro Molekül auf. Vorzugsweise weist die Verbindung der Formel (I) von 2 bis 14, vorzugsweise von 4 bis 12, insbesondere von 6 bis 10 Einheiten A pro Molekül auf.

Der Index n ist eine ganze Zahl ≥0, vorzugsweise von 0 bis 10, insbesondere von 0 bis 5. Der Index n ist also vorzugsweise eine ganze Zahl ausgewählt aus 0, 1, 2, 3, 4, 5, 6, 7, 8, 9 und 10. Bevorzugte Verbindungen der Formel (I) sind dadurch gekennzeichnet, dass für mindestens zwei Reste R gilt, dass n ungleich 0 ist. Es ist weiterhin bevorzugt, dass für mindestens einen der beiden Reste R des Rests -NR₂ gilt, dass n ungleich 0 ist.

Bevorzugt handelt es sich bei den speziellen propoxylierten Benzoldicarbonsäureamiden also um Verbindungen der Formel (I), 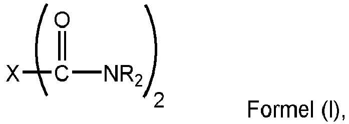 wobei
- X: ein Phenylenrest ist,
- R: jeweils unabhängig voneinander ausgewählt ist aus Resten der Formel (II), -[A-O]ₙ-H Formel (II),
- A: ein Propylenrest ist, und
- n: eine ganze Zahl ≥0, vorzugsweise von 0 bis 10, insbesondere von 0 bis 5 ist,
mit der Maßgabe, dass die Verbindung der Formel (I) von 2 bis 14, vorzugsweise 4 bis 12, insbesondere 6 bis 10 Einheiten A aufweist.

Die erfindungsgemäßen Verbindungen der Formel (I) liegen üblicherweise in Mischung vor, insbesondere da der Index n in den Resten R variieren kann und somit üblicherweise eine Molekulargewichtsverteilung vorliegt.

Ein weiterer Gegenstand der Erfindung ist daher auch eine Mischung umfassend oder bestehend aus Verbindungen der Formel (I), dadurch gekennzeichnet, dass die mittlere Anzahl der Einheiten A der Verbindung der Formel (I) von 2 bis 14, vorzugsweise von 4 bis 12, insbesondere von 6 bis 10 pro Molekül beträgt. Die mittlere Anzahl der Einheiten A pro Molekül der Verbindung der Formel (I) beträgt also beispielsweise 2, 3, 4, 5, 6, 7, 8, 9, 10, 11, 12, 13 oder 14. Die mittlere Anzahl der Einheiten A pro Molekül der Verbindung der Formel (I) kann auch nicht ganzzahlige Werte einnehmen. So nimmt die mittlere Anzahl der Einheiten A der Verbindung der Formel (I) in einer Mischung aus 1 mol einer Verbindung der Formel (I) mit 2 Einheiten A und 3 mol einer Verbindung der Formel (I) mit 3 Einheiten A den Wert 2,75 pro Molekül an. Unter der "Mischung umfassend oder bestehend aus Verbindungen der Formel (I)" wird hier ganz allgemein eine Zusammensetzung verstanden, die Verbindungen der Formel (I) umfasst bzw. aus diesen besteht. Der Begriff "Mischung" wird hier allerdings bevorzugt verwendet, um diese erfindungsgemäße Zusammensetzung von anderen erfindungsgemäßen Zusammensetzungen zu unterscheiden.

Ein weiterer Gegenstand der Erfindung sind daher auch Verbindungen der Formel (I) bzw. Mischungen umfassend oder bestehend aus Verbindungen der Formel (I), 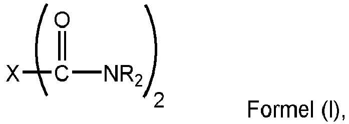 wobei
- X: ein Phenylenrest ist,
- R: jeweils unabhängig voneinander ausgewählt ist aus Resten der Formel (II), -[A-O]ₘ-H Formel (III),
- A: ein Propylenrest ist, und
- m: vorzugsweise von 0,5 bis 3,5, insbesondere von 1,0 bis 3,0, insbesondere 1,5 bis 2,5 beträgt.

Der Index m ergibt sich dabei aus der mittleren Anzahl der Einheiten A pro Molekül der Verbindung der Formel (I) dividiert durch die Anzahl der Reste R, also dividiert durch 4.

Die zahlenmittlere Molmasse Mₙ, gewichtsmittlere Molmasse M_{w} und Polydispersität der Verbindung der Formel (I) bzw. ihrer Mischungen ist prinzipiell beliebig. Es ist bevorzugt, dass die zahlenmittlere Molmasse Mₙ der Verbindung der Formel (I) bzw. ihrer Mischungen von 150 g/mol bis 6000 g/mol, bevorzugter von 200 g/mol bis 5000 g/mol, noch bevorzugter von 250 g/mol bis 2000 g/mol, besonders bevorzugt von 300 g/mol bis 1200 g/mol beträgt. Die zahlenmittlere Molmasse Mₙ wird dabei vorzugsweise mittel GPC bestimmt, wie in den Beispielen beschrieben.

Die Verbindung der Formel (I) bzw. ihrer Mischungen sind je nach Zusammensetzung und Molmasse flüssig, pastös oder fest. Ihrer Polydispersität ist in weiten Bereichen variabel. Es ist bevorzugt, dass die Polydispersität M_{w}/Mₙ nach GPC-Methode (bestimmt wie in den Beispielen beschrieben) von 1,0 bis 8,0, vorzugsweise von 1,1 bis 5,0, insbesondere von 1,2 bis 4,0 beträgt.

Die mittlere Zahl der OH-Gruppen pro Molekül der Verbindung der Formel (I) beträgt vorzugsweise 2 bis 4. Die OH-Zahl der Verbindung der Formel (I) bzw. ihrer Mischungen, gemessen wie in den Beispielen beschreiben, beträgt je nach Zusammensetzung bevorzugt zwischen 50 und 1000 mg KOH/g, bevorzugter zwischen 100 und 700 mg KOH/g, noch bevorzugter zwischen 250 und 600 mg KOH/g und ganz besonders bevorzugt zwischen 300 und 500 mg KOH/g.

Die Säurezahl der Verbindung der Formel (I) bzw. ihrer Mischungen, gemessen wie in den Beispielen beschreiben, beträgt von -1 bis 1 mg KOH/g, bevorzugt -0,5 bis 0,5 mg KOH/g, besonders bevorzugt 0 bis 0,5 mg KOH/g. Eine negative Säurezahl von -1 mg KOH/g ist gleichbedeutend mit der Alkalizahl von 1 mg KOH/g und bedeutet, dass die Probe noch einen Rest Alkalität aufweist

Verfahren zur Herstellung von Verbindungen der Formel (I) sind dem Fachmann bekannt. Besonders vorteilhaft ist allerdings das im Folgenden beschriebene Herstellverfahren.

Ein weiterer Gegenstand der Erfindung ist ein Verfahren zur Herstellung von einer oder mehreren Verbindungen der Formel (I), umfassend die Schritte:
a) Umsetzung mindestens einer aromatischen Verbindung ausgewählt aus der Gruppe bestehend aus Benzoldicarbonsäure, Benzoldicarbonsäureester und Benzoldicarbonsäureanhydrid mit mindestens einem Aminoalkohol ausgewählt aus der Gruppe bestehend aus Isopropanolamin und Diisopropanolamin, optional in Gegenwart eines alkalischen Katalysators, zu mindestens einem aromatischen Amid;
b) Umsetzung des mindestens einen aromatischen Amids mit Propylenoxid zu mindestens einer Verbindung der Formel (I).

Vorzugsweise ist das Verfahren weiterhin dadurch gekennzeichnet, dass
im Schritt a) mindestens ein Benzoldicarbonsäureester mit einem Aminoalkohol ausgewählt aus der Gruppe bestehend aus Isopropanolamin und Diisopropanolamin in Gegenwart eines alkalischen Katalysators aminolytisch zu dem korrespondierenden aromatischen Amid umgesetzt wird, wobei der aus dem Ester entstehende Alkohol destillativ entfernt wird und der Katalysator im entstandenen aromatischen Amid verbleibt;
im Schritt b) an die OH-Gruppen und ggf. NH-Gruppen des entstandenen aromatischen Amids Propylenoxid addiert wird, wobei bevorzugt der in Schritt a) verwendete alkalische Katalysator auch als Katalysator für die Alkoxylierungsreaktion dient;
in einem optionalen im Schritt c) das Reaktionsprodukt aus Schritt b) mit einer Säure neutralisiert, filtriert und mit Additiven wie etwa einem Antioxidanz versetzt wird.

In einer besonders bevorzugten Ausführungsform finden die Schritte a) und b) direkt hintereinander im selben Reaktionsbehälter statt. Dies hat den Vorteil, dass die heiße Schmelze des aromatischen Amids aus Schritt a) nicht abgelassen werden muss und somit nicht zu einer festen kristallinen oder glasartigen Masse erstarrt, sondern direkt im Schritt b) weiterverarbeitet werden kann. Dieses Vorgehen erspart Zeit, die für das Wiedereinfüllen des festen aromatischen Amids in den Alkoxylierungsreaktor für Schritt b) notwendig wäre, und führt zudem zu farblich helleren, höherwertigen Produkten, da der Kontakt mit Luftsauerstoff vermieden wird und der gesamte Prozess unter Inertgasatmosphäre stattfinden kann. Möglich wird dieses vorteilhafte erfindungsgemäße Verfahren durch die geschickte Verwendung eines alkalischen Katalysators, der sowohl die Amidierung im Schritt a) als auch die Alkoxylierung im Schritt b) effektiv katalysiert.

Das erfindungsgemäße Verfahren ermöglicht es auf einfache Weise, diverse aromatische Verbindungen ausgewählt aus der Gruppe bestehend aus Benzoldicarbonsäuren, Benzoldicarbonsäureestern und Benzoldicarbonsäureanhydrid umzusetzen.

Die Kettenlänge der Reste R der Verbindung der Formel (I) kann in weiten Bereichen variiert werden. Die mittlere Anzahl der Reste R ist gezielt über die Wahl des Aminoalkohols einstellbar und eröffnet eine große strukturelle Vielfalt. Auf diesem Wege sind anwendungstechnisch bedeutsame Eigenschaften wie Viskosität und OH-Funktionalität frei wählbar.

Die durch das erfindungsgemäße Verfahren erhältliche Verbindungen der Formel (I) sind vorzugsweise im Wesentlichen frei von restlichen Aminoalkoholen und enthalten vorzugsweise im Wesentlichen keine freien Polyetheranteile. Vorzugsweise sind im Wesentlichen alle Polyether chemisch über eine Amidgruppe an das aromatische Gerüst der Verbindung der Formel (I) angebunden.

Es ist bevorzugt, dass in Schritt a) des Verfahrens zur Herstellung von einer oder mehreren Verbindungen der Formel (I) als aromatische Verbindung Phthalsäuredimethylester (Dimethylphthalat) und/oder Isophthalsäuredimethylester eingesetzt wird.

Es ist dabei besonders bevorzugt, dass in Schritt a) des Verfahrens zur Herstellung von einer oder mehreren Verbindungen der Formel (I) als aromatische Verbindung Dimethylphthalat eingesetzt wird.

Als alkalische Katalysatoren können in Schritt a) können prinzipiell alle Alkalihydroxide und Alkalialkoholate verwendet werden.

Es ist bevorzugt, dass in Schritt a) des Verfahrens zur Herstellung von einer oder mehreren Verbindungen der Formel (I) als alkalischer Katalysator wahlweise Natriummethanolat, Kaliummethanolat, NaOH oder KOH, bevorzugt Natriummethanolat oder Kaliummethanolat, besonders bevorzugt Natriummethanolat eingesetzt wird. Bevorzugt eingesetzt werden Natriummethanolat und Kaliummethanolat - entweder als Feststoff oder gelöst in Methanol. Besonders bevorzugt ist eine Lösung von Natriummethanolat in Methanol. ganz besonders bevorzugt ist eine Lösung aus 30 Massenteilen Natriummethanolat und 70 Massenteilen Methanol.

Es ist bevorzugt, dass im Schritt a) des Verfahrens zur Herstellung von einer oder mehreren Verbindungen der Formel (I) die aromatische Verbindung ausgewählt aus der Gruppe bestehend aus Benzoldicarbonsäure, Benzoldicarbonsäureester und Benzoldicarbonsäureanhydrid mit dem Katalysator im Reaktor vorgelegt und das Alkanolamin zugegeben wird. Die Umsetzung findet nach Inertisierung mit Stickstoff bei 50 °C bis 200 °C, bevorzugt 70 °C bis 150 °C, besonders bevorzugt bei 90 °C bis 130 °C statt. Vorzugsweise wird dabei der bei der Umsetzung abgespaltene Alkohol bzw. das abgespaltene Wasser im Vakuum oder bei Normaldruck destillativ aus dem Reaktionsgemisch entfernt. Dabei wird das mit der Katalysatorlösung eingebrachte Methanol ebenfalls entfernt.

Es ist bevorzugt, dass im Schritt a) des Verfahrens zur Herstellung von einer oder mehreren Verbindungen der Formel (I) das Alkanolamin (ausgewählt aus der Gruppe bestehend aus Isopropanolamin und Diisopropanolamin) in einem molaren Verhältnis zur aromatischen Verbindung (ausgewählt aus der Gruppe bestehend aus Benzoldicarbonsäure, Benzoldicarbonsäureester und Benzoldicarbonsäureanhydrid) von 0,8:1 bis 1,3:1, vorzugsweise von 0,9:1 bis 1,1:1, insbesondere 1:1 einzusetzen.

Es ist bevorzugt, dass im Schritt a) des Verfahrens zur Herstellung von einer oder mehreren Verbindungen der Formel (I) der alkalische Katalysator in einer Konzentration von 0,2 bis 5 Gew.-%, vorzugsweise von 0,5 bis 4 Gew.-%, insbesondere von 1,0 bis 2,5 Gew.-% bezogen auf Einwaage der aromatischen Verbindung ausgewählt aus der Gruppe bestehend aus Benzoldicarbonsäure, Benzoldicarbonsäureester und Benzoldicarbonsäureanhydrid eingesetzt wird.

Das Ende der Reaktion ist daran zu erkennen, dass kein Destillat (Alkohol bzw. Wasser) mehr fließt. Es schließt sich eine Nachreaktion von üblicherweise 0,5 h bis 2,5 h bei 90 °C bis 130 °C an. Der alkalische Katalysator verbleibt vorzugsweise in der Reaktionsmischung.

Schritt a) wird vorzugsweise in dem Reaktor durchgeführt, in dem im Anschluss Schritt b) (also die Alkoxylierungsreaktion) durchgeführt wird. Alternativ kann das Reaktionsprodukt aus Schritt a) (also das aromatische Amid) als Schmelze abgelassen und in flüssiger oder fester Form in einen geeigneten Alkoxylierungsreaktor überführt werden.

Das aromatische Amid aus Schritt a) dient im Schritt b) als Startverbindung für die Umsetzung Propylenoxid. Unter Ringöffnung und vorzugsweise in Gegenwart eines geeigneten Katalysators wird die Propylenoxid, in einer Additionsreaktion an die reaktiven OH-Gruppen des aromatischen Amids (D) addiert. Je nach Reaktionsbedingungen kann die Addition auch zusätzlich an der NH-Gruppe des Amids erfolgen, sofern solche vorhanden sind.

Durch das erfindungsgemäße Verfahren lassen sich Polyetherketten am aromatischen Amid aufgebaut werden. Die Molmassen der Polyetherreste können nach dem erfindungsgemäßen Verfahren in weiten Grenzen variiert und gezielt und reproduzierbar über das Molverhältnis des Propylenoxids OH-Gruppen und ggfs. NH-Gruppen des mindestens einen vorgelegten Amids (D) gesteuert werden.

Es ist dabei bevorzugt, dass im Schritt b) des Verfahrens zur Herstellung von einer oder mehreren Verbindungen der Formel (I) das Stoffmengenverhältnis von Propylenoxid zur Gesamtstoffmenge aller aromatischen Amide von 1 bis 10, vorzugsweise von 2 bis 8, insbesondere von 3 bis 6 beträgt.

Vor der Zugabe Propylenoxids wird der mit dem aromatischen Amid und ggf. Katalysator teilweise befüllte Reaktor vorzugsweise mit Stickstoff inertisiert. Dies geschieht beispielsweise durch mehrfaches abwechslungsweises Evakuieren und Zuführen von Stickstoff. Es ist vorteilhaft, den Reaktor nach dem letzten Aufdrücken von Stickstoff auf unter 200 mbar zu evakuieren. Die Addition der ersten Menge an Propylenoxid findet somit vorzugsweise in den evakuierten Reaktor statt. Die Dosage des Propylenoxids erfolgt unter Rühren und ggf. Kühlen, um die freiwerdende Reaktionswärme abzuführen und die vorgewählte Reaktionstemperatur einzuhalten.

Es ist bevorzugt, dass die Reaktionstemperatur in Schritt b) des Verfahrens zur Herstellung von einer oder mehreren Verbindungen der Formel (I) von 80 °C bis 200 °C, vorzugsweise von 90 °C bis 160 °C, insbesondere von 100 °C bis 160 °C beträgt.

Es ist dabei bevorzugt, dass der Innendruck (absolut) des Reaktors in Schritt b) des Verfahrens zur Herstellung von einer oder mehreren Verbindungen der Formel (I) von 0,2 bar bis 100 bar, vorzugsweise von 0,5 bar bis 20 bar, insbesondere von 1 bar bis 6 bar beträgt.

Besonders bevorzugt wird die Umsetzung in Schritt b) des Verfahrens zur Herstellung von einer oder mehreren Verbindungen der Formel (I) bei einer Temperatur von 100 °C bis 160 °C und einem Druck von 1 bar bis 6 bar durchgeführt.

Sofern in Schritt a) ein alkalischer Katalysator eingesetzt wurde, ist es bevorzugt, dass dieser auch in Schritt b) als alkalischer Katalysator dient. Es können allerdings auch andere Katalysatoren eingesetzt werden, wie beispielsweise Doppelmetallcyanid-Katalysatoren (DMC-Katalysatoren).

Die Umsetzung in Schritt b) des Verfahrens zur Herstellung von einer oder mehreren Verbindungen der Formel (I) kann optional in einem geeigneten Lösemittel durchgeführt werden. Nach Beendigung der Propylenoxid-Addition folgt vorzugsweise eine Nachreaktion zur Vervollständigung des Umsatzes. Die Nachreaktion kann z.B. durch Weiterreaktion bei Reaktionsbedingungen ohne Zugabe von Edukten durchgeführt werden. Nicht abreagiertes Propylenoxid und weitere flüchtige Bestandteile können zum Schluss durch Vakuumdestillation, Wasserdampf- oder Gasstrippen oder andere Methoden der Desodorierung entfernt werden.

In einem optionalen mittelbar oder unmittelbar auf Schritt b) folgenden Schritt c) des Verfahrens zur Herstellung von einer oder mehreren Verbindungen der Formel (I) kann das Umsetzungsprodukt aus Schritt b) mit einer beliebigen Säure neutralisiert werden. Beispielhaft seien genannt Phosphorsäure, Methansulfonsäure, p-Toluolsulfonsäure, Schwefelsäure oder Carbonsäuren wie Essigsäure und Milchsäure. Bevorzugt ist der Einsatz wässriger Phosphorsäure und Milchsäure. Die Einsatzmenge der jeweiligen Säure richtet sich nach der zuvor verwendeten Menge an basischem Katalysator. Das neutralisierte Polyol wird bei Bedarf anschließend in einer Vakuumdestillation bei <100 mbar und 80 °C bis 130 °C trocken destilliert. Das neutralisierte Produkt wird abschließend bei <100 °C filtriert, um ausgefällte Salze zu entfernen. Die erfindungsgemäßen Endprodukte haben einen Wassergehalt von <0,2 %, eine Säurezahl von <0,5 mg KOH/g und sind praktisch phosphatfrei.

Der Verbindung der Formel (I) können optional vor, während oder nach der optionalen Neutralisation Additive zugefügt werden wie z.B. Antioxidanzien. Hierzu gehören sterisch gehinderte Phenol, u.a. bekannt unter den Handelsnamen Irganox 1135, Irganox 1010, Irganox 1076, Anox 20.

Als Reaktoren für das erfindungsgemäße Verfahren zur Herstellung von einer oder mehreren Verbindungen können prinzipiell alle geeigneten Reaktortypen zum Einsatz kommen, die die Reaktion und ihre eventuell vorhandene Wärmetönung beherrschen lassen. Die Reaktionsführung kann in verfahrenstechnisch bekannter Art und Weise kontinuierlich, semi-kontinuierlich oder auch batchweise erfolgen und lässt sich flexibel auf die vorhandenen produktionstechnischen Einrichtungen abstimmen. Neben herkömmlichen Rührkesselreaktoren können auch Strahlschlaufenreaktoren mit Gasphase und internen Wärmetauscherrohren, wie in WO 01/062826 beschrieben, verwendet werden. Darüber hinaus können gasphasenfreie Loop-Reaktoren eingesetzt werden.

Wie bereits eingangs erläutert, werden die Verbindungen der Formel (I) vorzugweise als Polyol-Komponente oder als Bestandteil einer Polyol-Komponente einer Zusammensetzung zur Herstellung von Polyurethanschaum verwendet.

Ein weiterer Gegenstand der Erfindung ist daher auch eine Zusammensetzung zur Herstellung von Polyurethanschaum, umfassend mindestens eine Polyisocyanat-Komponente, mindestens eine Polyol-Komponente, optional mindestens einen Katalysator, der die Ausbildung einer Urethan- oder Isocyanurat-Bindung katalysiert, und optional mindestens ein Treibmittel, dadurch gekennzeichnet, dass mindestens eine Polyol-Komponente ausgewählt ist aus Verbindungen der Formel (I) oder aus Mischungen von Verbindungen der Formel (I).

Die Zusammensetzung kann also eine oder mehrere Polyisocyanat-Komponenten, eine oder mehrere Polyol-Komponenten, optional einen oder mehrere Katalysatoren, die die Ausbildung einer Urethan- oder Isocyanurat-Bindung katalysieren, und optional ein oder mehrere Treibmittel, umfassen.

Bevorzugte erfindungsgemäße Zusammensetzungen, die zur Herstellung von Polyurethan- oder Polyisocyanurat-Hartschaumstoffen geeignet sind, enthalten zumindest eine Isocyanatkomponente, zumindest eine Polyolkomponente, zumindest einen Schaumstabilisator, zumindest einen Urethan- und/oder Isocyanurat-Katalysatoren, Wasser und/oder Treibmittel, und optional zumindest ein Flammschutzmittel und/oder weitere Additive, und zeichnen sich dadurch aus, dass zumindest Verbindungen der Formel (I) enthalten ist.

Eine bevorzugte erfindungsgemäße Zusammensetzung enthält also die folgenden Bestandteile:
(a) eine oder mehrere Polyol-Komponenten, enthaltend eine oder mehrere Verbindung der Formel (I)
(b) eine oder mehrere Polyisocyanat-Komponenten
(c) optional ein oder mehrere Katalysatoren
(d) optional einen oder mehrere Schaumstabilisatoren
(e) optional ein oder mehrere Treibmitteln
(f) optional ein oder mehreren Additive, vorzugsweise ausgewählt aus der Gruppe bestehend aus Füllstoffen und Flammschutzmitteln.

Eine besonders bevorzugte Zusammensetzung enthält die folgenden Bestandteile:
(a) eine oder mehrere Polyol-Komponenten, enthaltend eine oder mehrere Verbindung der Formel (I)
(b) eine oder mehrere Polyisocyanat-Komponenten
(c) ein oder mehrere Katalysatoren
(d) optional einen oder mehrere Schaumstabilisatoren
(e) ein oder mehrere Treibmitteln
(f) optional ein oder mehreren Additive, vorzugsweise ausgewählt aus der Gruppe bestehend aus Füllstoffen und Flammschutzmitteln.

In der erfindungsgemäßen Zusammensetzung beträgt der Massenanteil an Verbindungen der Formel (I) bezogen auf 100 Massenteile Polyol-Komponente (a) vorzugsweise von 5 bis 100 pphp, bevorzugt von 10 bis 70 pphp und besonders bevorzugt von 15 bis 50 pphp.

Dabei steht pphp für "parts per hundred polyol" bzw. "parts per hundred parts of polyol" und bedeutet Massenteile bezogen auf 100 Massenteile des Polyols (also der Polyol-Komponente).

Es ist bevorzugt, dass die erfindungsgemäße Zusammensetzung dadurch gekennzeichnet ist, dass der Massenanteil aller Verbindungen der Formel (I) bezogen auf die Gesamtmasse der Zusammensetzung von 3 % bis 40 %, vorzugsweise von 5 % bis 30 %, insbesondere von 10 % bis 20 % beträgt.

Es ist bevorzugt, dass die erfindungsgemäße Zusammensetzung dadurch gekennzeichnet ist, dass der Massenanteil aller Verbindungen der Formel (I) bezogen auf die Gesamtmasse aller Polyol-Komponenten von 5 % bis 65 %, vorzugsweise von 10 % bis 50%, insbesondere von 15 % bis 45 % beträgt.

Neben der obligaten Verbindung der Formel (I) können noch weitere Polyol-Komponenten enthalten sein.

Als Polyol-Komponente (a) werden eine oder mehrere Verbindungen mit OH-Gruppen, SH-Gruppen, NH-Gruppen und/oder NH₂-Gruppen, mit einer Funktionalität von 1,8 bis 8 eingesetzt. Dabei umfasst die Polyol-Komponente mindestens eine Verbindung mit mindestens zwei Isocyanat-reaktiven Gruppen, ausgewählt aus OH-Gruppen, SH-Gruppen, NH-Gruppen und/oder NH₂-Gruppen, insbesondere OH-Gruppen.

Eine Funktionalität von z.B. 1,8 kann sich dadurch ergeben, dass mindestens eine Verbindung mit einer höheren Funktionalität z.B. größer oder gleich 2, mit mindestens einer Verbindung mit einer Funktionalität von z.B. 1 gemischt wird. Dies kann insbesondere geschehen, wenn ein Polyisocyanat-Komponente (b) mit einer Funktionalität größer 2 oder zusätzliche Vernetzer als optionale Additive (f) eingesetzt werden.

Geeignete Verbindungen im Sinne dieser Erfindung sind alle organischen Substanzen mit OH-Gruppen, SH-Gruppen, NH-Gruppen und/oder NH₂-Gruppen, insbesondere OH-Gruppen, sowie deren Mischungen mit einer Funktionalität von 1,8 bis 8.

Entsprechende Verbindungen, die üblicherweise bei der Herstellung von PU-Schäume eingesetzt werden können, sind dem Fachmann bekannt und beispielsweise beschrieben im "Kunststoffhandbuch, Band 7, Polyurethane", Carl Hanser Verlag, 3. Auflage 1993, Kapitel 3.1.

Üblicherweise kommen Verbindungen mit OH-Zahlen im Bereich von 10 bis 1200 mg KOH/g zum Einsatz. Die OH-Zahl wird dabei vorzugsweise entweder gemäß der Norm DIN EN ISO 4629-1:2016-12 (ohne Katalysator) oder gemäß der Norm DIN EN ISO 4629-2:2016-12 (mit Katalysator) bestimmt.

Es ist bevorzugt, dass die Polyole bzw. die Polyol-Komponente ein zahlengemitteltes Molekulargewicht von 500 bis 15000 g/mol aufweist. Das zahlengemittelte Molekulargewicht kann dabei beispielsweise mittels Gel-Permeations-chromatographie (GPC) bestimmt werden, bevorzugt gemäß der Norm DIN EN ISO 13885-1:2021-11 (THF als Elutionsmittel), gemäß der Norm DIN EN ISO 13885-2:2021-11 (Acrylamid als Elutionsmittel) oder gemäß der Norm ISO 13885-3:2020-07 (Wasser als Elutionsmittel), besonders bevorzugt gemäß DIN EN ISO 13885-1:2021-11 (THF als Elutionsmittel).

Üblicherweise besitzen also die Polyole bzw. die Polyol-Komponente eine Funktionalität von 1,8 bis 8 und zahlengemittelte Molekulargewichte im Bereich von 500 bis 15000 g/mol. Üblicherweise kommen die Polyole mit OH-Zahlen im Bereich von 10 bis 1200 mg KOH/g zum Einsatz

Besonders bevorzugte Verbindungen sind alle zur Herstellung von Polyurethansystemen, insbesondere Polyurethanschaumstoffen üblicherweise verwendeten Polyetherpolyole und Polyesterpolyole.

Zudem können Polyetherpolycarbonatpolyole, auf natürlichen Ölen basierende Polyole (natural oil based polyols, NOPs; z.B. beschrieben in WO 2005/033167, US 2006/0293400, WO 2006/094227, WO 2004/096882, US 2002/0103091, WO 2006/116456, EP 1678232), Füllkörperpolyole, prepolymerbasierte Polyole und/oder Recyclingpolyole eingesetzt werden.

Recyclingpolyole sind Polyole, die aus dem chemischen Recycling, zum Beispiel durch Solvolyse, wie beispielsweise Glykolyse, Hydrolyse, Acidolyse oder Aminolyse, von Polyurethanen erhalten werden. Der Einsatz von Recyclingpolyolen stellt eine besonders bevorzugte Ausführungsform der Erfindung dar.

Als Polyolkomponente a) geeignete Polyole im Sinne der vorliegenden Erfindung sind also alle organischen Substanzen mit einer oder mehreren gegenüber Isocyanaten reaktiven Gruppen, vorzugsweise OH-Gruppen, sowie deren Zubereitungen. Bevorzugte Polyole sind alle zur Herstellung von PolyurethanSystemen, insbesondere Polyurethan-Beschichtungen, Polyurethan-Elastomeren oder auch Schaumstoffen; üblicherweise verwendeten Polyetherpolyole und/oder Polyesterpolyole und/oder hydroxylgruppenhaltigen aliphatischen Polycarbonate, insbesondere Polyetherpolycarbonatpolyole und/oder Polyole natürlicher Herkunft, sogenannte "natural oil based polyols" (NOPs). Üblicherweise besitzen die Polyole eine Funktionalität von 1.8 bis 8 und zahlengemittelte Molekulargewichte im Bereich von 500 bis 15000. Üblicherweise kommen die Polyole mit OH-Zahlen im Bereich von 10 bis 1200 mg KOH/g zum Einsatz.

Polyetherpolyole können nach bekannten Verfahren hergestellt werden, beispielsweise durch anionische Polymerisation von Alkylenoxiden in Gegenwart von Alkalihydroxiden, Alkalialkoholaten oder Aminen als Katalysatoren und unter Zusatz mindestens eines Startermoleküls, dass bevorzugt 2 oder 3 reaktive Wasserstoffatome gebunden enthält oder durch kationische Polymerisation von Alkylenoxiden in Gegenwart von Lewis-Säuren wie beispielsweise Antimonpentachlorid oder Bortrifluorid-Etherat oder durch Doppelmetallcyanidkatalyse. Geeignete Alkylenoxide enthalten 2 bis 4 Kohlenstoffatome im Alkylenrest. Beispiele sind Tetrahydrofuran, 1,3-Propylenoxid, 1,2- bzw. 2,3-Butylenoxid; vorzugsweise werden Ethylenoxid und 1,2-Propylenoxid eingesetzt. Die Alkylenoxide können einzeln, kumulativ, blockweise, alternierend nacheinander oder als Mischungen verwendet werden. Als Startmoleküle kommen insbesondere Verbindungen mit mindestens 2, vorzugsweise 2 bis 8 Hydroxylgruppen oder mit mindestens zwei primären Aminogruppen im Molekül zum Einsatz. Als Startermoleküle eingesetzt werden können z.B. Wasser, 2-, 3- oder 4-wertige Alkohole wie Ethylenglykol, Propandiol-1,2 und -1,3, Diethylenglykol, Dipropylenglykol, Glycerin, Trimethylolpropan, Pentaerythrit, Rizinusöl usw., höhere polyfunktionelle Polyole, insbesondere Zuckerverbindungen wie beispielsweise Glucose, Sorbit, Mannit und Saccharose, mehrwertige Phenole, Resole, wie z.B. oligomere Kondensationsprodukte aus Phenol und Formaldehyd und Mannich-Kondensate aus Phenolen, Formaldehyd und Dialkanolaminen sowie Melamin, oder Amine wie Anilin, EDA, TDA, MDA und PMDA, besonders bevorzugt TDA und PMDA. Die Wahl des geeigneten Startermoleküls ist abhängig von dem jeweiligen Anwendungsgebiet des resultierenden Polyetherpolyols bei der Polyurethanherstellung.

Polyesterpolyole basieren auf Estern mehrwertiger aliphatischer oder aromatischer Carbonsäuren, bevorzugt mit 2 bis 12 Kohlenstoffatomen. Beispiele für aliphatische Carbonsäuren sind Bernsteinsäure, Glutarsäure, Adipinsäure, Korksäure, Azelainsäure, Sebacinsäure, Decandicarbonsäure, Maleinsäure und Fumarsäure. Beispiele für aromatische Carbonsäuren sind Phthalsäure, Isophthalsäure, Terephthalsäure und die isomeren Naphthalindicarbonsäuren. Die Polyesterpolyole werden durch Kondensation dieser mehrwertigen Carbonsäuren mit mehrwertigen Alkoholen, vorzugsweise von Diolen oder Triolen mit 2 bis 12, besonders bevorzugt mit 2 bis 6 Kohlenstoffatomen, bevorzugt Trimethylolpropan und Glycerin erhalten.

In einer besonders bevorzugten Ausführungsform werden Polyesterpolyole auf Basis von aromatischen Carbonsäuren in mehr als 50 pphp, bevorzugt mehr als 70 pphp eingesetzt, bezogen auf 100 Massenteile Polyol-Komponente.

In einer weiteren ganz besonders bevorzugten Ausführungsform werden keine Polyole auf Basis von Phenol-Harzen, hergestellt aus Novolaken und Alkylenoxiden, und keine Polyole auf Basis von aromatischen Amin-Polyolen, hergestellt durch Alkoxylierung von aromatischen Aminen, eingesetzt, was bedeutet, dass in dieser bevorzugten Ausführungsform weniger als 20 pphp, vorzugsweise weniger als 10 pphp, insbesondere weniger als 2 pphp, und am vorteilhaftesten überhaupt keine Polyole auf Basis von Phenol-Harzen, hergestellt aus Novolaken und Alkylenoxiden, und überhaupt keine Polyole auf Basis von aromatischen Amin-Polyolen, hergestellt durch Alkoxylierung von aromatischen Aminen, eingesetzt werden.

Polyetherpolycarbonatpolyole sind Polyole, welche Kohlenstoffdioxid als Carbonat gebunden enthalten. Da Kohlenstoffdioxid bei vielen Prozessen in der chemischen Industrie in großen Mengen als Nebenprodukt entsteht, ist die Verwendung von Kohlendioxid als Comonomer in Alkylenoxid-Polymerisationen aus kommerzieller Sicht von besonderem Interesse. Ein teilweiser Ersatz von Alkylenoxiden in Polyolen durch Kohlendioxid hat das Potential, die Kosten für die Herstellung von Polyolen deutlich zu senken. Außerdem ist die Verwendung von CO₂ als Comonomer ökologisch sehr vorteilhaft, da diese Reaktion die Umsetzung eines Treibhausgases zu einem Polymer darstellt. Die Herstellung von Polyetherpolycarbonatpolyolen durch Anlagerung von Alkylenoxiden und Kohlendioxid an H-funktionelle Startsubstanzen unter Verwendung von Katalysatoren ist seit langem bekannt. Verschiedene Katalysatorsysteme können hierbei zum Einsatz kommen: Die erste Generation stellten heterogene Zink- oder Aluminiumsalze dar, wie sie beispielsweise in US-A 3900424 oder US-A 3953383 beschrieben sind. Des Weiteren sind mono- und binukleare Metallkomplexe zur Copolymerisation von CO₂ und Alkylenoxiden erfolgreich eingesetzt worden (WO 2010/028362, WO 2009/130470, WO 2013/022932 oder WO 2011/163133). Die wichtigste Klasse von Katalysatorsystemen für die Copolymerisation von Kohlenstoffdioxid und Alkylenoxiden stellen die Doppelmetallcyanidkatalysatoren, auch als DMC-Katalysatoren bezeichnet, dar (US-A 4500704, WO 2008/058913). Geeignete Alkylenoxide und H-funktionelle Startsubstanzen sind solche, die auch zur Herstellung von carbonatfreien Polyetherpolyolen - wie oben beschrieben - eingesetzt werden.

Polyole auf Basis nachwachsender Rohstoffe "Natural oil based polyols" (NOPs) zur Herstellung von Polyurethanschäumen sind mit Blick auf die langfristig begrenzte Verfügbarkeit fossiler Ressourcen, namentlich Öl, Kohle und Gas, und vor dem Hintergrund steigender Rohölpreise von zunehmendem Interesse und bereits vielfach in solchen Anwendungen beschrieben (WO 2005/033167; US 2006/0293400, WO 2006/094227, WO 2004/096882, US 2002/0103091, WO 2006/116456 und EP 1678232). Mittlerweile sind auf dem Markt eine Reihe dieser Polyole von verschiedenen Herstellern verfügbar (WO 2004/020497, US 2006/0229375, WO 2009/058367). In Abhängigkeit vom Basis-Rohstoff (z.B. Sojabohnenöl, Palmöl oder Rizinusöl) und die daran angeschlossene Aufarbeitung ergeben sich Polyole mit unterschiedlichem Eigenschaftsbild. Hierbei können im Wesentlichen zwei Gruppen unterschieden werden: a) Polyole auf Basis nachwachsender Rohstoffe, die so weit modifiziert werden, dass sie zu 100 % zur Herstellung von Polyurethanen eingesetzt werden können (WO 2004/020497, US 2006/0229375); b) Polyole auf Basis nachwachsender Rohstoffe, die bedingt durch ihre Aufarbeitung und Eigenschaften nur zu einem gewissen Anteil das petrochemisch basierte Polyol ersetzen können (WO2009/058367) .

Eine weitere Klasse von einsetzbaren Polyolen stellen die sogenannten Füllkörperpolyole (Polymerpolyole) dar. Diese zeichnen sich dadurch aus, dass sie feste organische Füllstoffe bis zu einem Feststoffgehalt von 40 % oder mehr in disperser Verteilung enthalten. Einsetzbar sind unter anderem SAN-, PHD- und PIPA-Polyole. SAN-Polyole sind hochreaktive Polyole, welche ein Copolymer auf der Basis von Styrol/Acrylnitril (SAN) dispergiert enthalten. PHD-Polyole sind hochreaktive Polyole, welche Polyharnstoff ebenfalls in dispergierter Form enthalten. PIPA-Polyole sind hochreaktive Polyole, welche ein Polyurethan, beispielsweise durch in situ-Reaktion eines Isocyanats mit einem Alkanolamin in einem konventionellen Polyol gebildet, in dispergierter Form enthalten.

Eine weitere Klasse von einsetzbaren Polyolen sind solche, die als Prepolymere durch Umsetzung von Polyol mit Isocyanat in einem Molverhältnis von vorzugsweise 100 zu 1 bis 5 zu 1, bevorzugt 50 zu 1 bis 10 zu 1, erhalten werden. Solche Prepolymere werden vorzugsweise gelöst in Polymer angesetzt, wobei das Polyol bevorzugt dem zur Herstellung der Prepolymeren eingesetzten Polyol entspricht.

Als Polyisocyanat-Komponente (b) werden im Allgemeinen ein oder mehrere Polyisocyanate mit zwei oder mehr Isocyanat-Gruppen eingesetzt. Geeignete Polyisocyanate im Sinne dieser Erfindung sind alle organischen Isocyanate mit zwei oder mehr Isocyanat-Gruppen, insbesondere die an sich bekannten aliphatischen, cycloaliphatischen, arylaliphatischen und vorzugsweise aromatischen mehrfunktionellen Isocyanate.

Besonders bevorzugt werden Isocyanate in einem Bereich von 60 bis 200 mol% relativ zu der Summe der isocyanatverbrauchenden Komponenten eingesetzt.

Beispielhaft genannt werden können hier Alkylendiisocyanate mit 4 bis 12 Kohlenstoffatomen im Alkylenrest, wie 1,12-Dodecandiisocyanat, 2-Ethyl-tetramethylen-1,4-diisocyanat, 2-Methyl-pentamethylen-1,5-diisocyanat, Tetramethylen-1,4-diisocyanat, Pentamethylendiisocyanat (PDI) und vorzugsweise Hexamethylen-1,6-diisocyanat (HMDI), cycloaliphatische Diisocyanate, wie Cyclohexan-1,3- und -1-4-diisocyanat sowie die entsprechenden Isomerengemische, 4,4'-Methylendicyclohexyldiisocyanat (H12MDI), Isophorondiisocyanat (IPDI), 2,4- und 2,6-Methylcyclohexyldiisocyanat sowie die entsprechenden Isomerengemische und vorzugsweise aromatische Di- und Polyisocyanate, wie 2,4- und 2,6-Toluoldiisocyanat (TDI) sowie die entsprechenden Isomerengemische, Naphthylendiisocyanat, Diethyltoluoldiisocyanat, 4,4'- oder 2,2'- oder 2,4'-Diphenylmethandiisocyanat (MDI) und Polymethylenpolyphenyl-polyisocyanate (PMDI, "polymeres MDI"). Die organischen Polyisocyanate können einzeln oder in Form ihrer Mischungen eingesetzt werden. Ebenso können entsprechende "Oligomere" der Diisocyanate eingesetzt werden, wie z.B. das IPDI-Trimer auf Basis des Isocyanurates, Biurete oder Urethdione. Des Weiteren ist der Einsatz von Prepolymeren auf Basis der oben genannten Isocyanate möglich. Besonders geeignet ist das als "polymeres MDI" (auch als "crude MDI" oder "Roh-MDI" bezeichnet) bekannte Gemisch aus MDI und höher kondensierten Analoga mit einer mittleren Funktionalität von 2 bis 4, sowie die verschiedenen Isomere des TDI in reiner Form oder als Isomerengemisch. Es ist auch möglich, Isocyanate einzusetzen, die durch den Einbau von Urethan-, Uretdion-, Isocyanurat-, Allophanat- und anderen Gruppen modifiziert wurden, sogenannte modifizierte Isocyanate. Besonders geeignete organische Polyisocyanate und daher besonders bevorzugt angewendet werden verschiedene Isomere des Toluoldiisocyanat (2,4- und 2,6-Toluoldiisocyanat (TDI), in reiner Form oder als Isomerengemische unterschiedlicher Zusammensetzung), 4,4'-Diphenylmethandiisocyanat (MDI), das so genannte "crude MDI" oder "polymere MDI" (enthält neben dem 4,4'- auch die 2,4'- und 2,2'-Isomeren des MDI und höherkernige Produkte) sowie das als "pure MDI" bezeichnete zweikernige Produkt aus überwiegend 2,4'- und 4,4'-lsomerengemischen bzw. deren Prepolymeren. Beispiele für besonders geeignete Isocyanate sind auch z.B. in EP 1712578, EP 1161474, WO 00/58383, US 2007/0072951, EP 1678232 und der WO 2005/085310 aufgeführt, auf die hier in vollem Umfang Bezug genommen wird.

Ein bevorzugtes Verhältnis von Polyisocyanat-Komponente (b) (also der Gesamtheit aller Polyisocyanat-Komponente) und Polyol-Komponente (a) (also der Gesamtheit aller Polyol-Komponenten), ausgedrückt als Index der Formulierung (auch als Isocyanat-Index der Formulierung bezeichnet), d.h. als stöchiometrisches Verhältnis von Isocyanat-Gruppen gegenüber isocyanat-reaktiven Gruppen (z.B. OH-Gruppen, NH-Gruppen) multipliziert mit 100, liegt im Bereich von 10 bis 1000, bevorzugt 40 bis 600. Ein Index von 100 steht für ein molares Verhältnis der reaktiven Gruppen von 1 zu 1.

In einer bevorzugten Ausführungsform der Erfindung ist der Index der Formulierung im Bereich 100 bis 250, besonders bevorzugt 105 bis 200.

In einer alternativen bevorzugten Ausführungsform der Erfindung ist der Index der Formulierung im Bereich 150 bis 550, besonders bevorzugt 200 bis 500. Das heißt, in einer bevorzugten Ausführungsform ist ein deutlicher Überschuss an Isocyanat-Gruppen zu Isocyanat-reaktiven Gruppen vorhanden. Dadurch kommt es zur Trimerisierungsreaktionen der Isocyanate, die somit Isocyanurate bilden. Diese Schaum-Typen werden auch als Polyisocyanurat (PIR)-Schäume bezeichnet und zeichnen sich durch ein verbessertes Brandverhalten, also schlechteres Brennen, aus. Diese Schaum-Typen sind ein bevorzugter Gegenstand der Erfindung. Besonders bevorzugt ist dabei als Polyol-Komponente (a) ein oder mehrere Polyesterpolyole umfasst.

Geeignete Katalysatoren (c), die für die Herstellung von Polyurethanen, insbesondere PU-Schäumen, einsetzbar sind, sind dem Fachmann aus dem Stand der Technik bekannt. Im Sinne der vorliegenden Erfindung sind alle Verbindungen einsetzbar, die in der Lage sind, die Reaktion von Isocyanat-Gruppen mit OH-, NH-, oder anderen isocyanat-reaktiven Gruppen, sowie die Reaktion von Isocyanat-Gruppen untereinander zu katalysieren.

Hierbei kann auf die üblichen aus dem Stand der Technik bekannten Katalysatoren zurückgegriffen werden, umfassend z.B. Amine (cyclische oder acyclische; Monoamine, Diamine, Oligomere mit einer oder mehreren Aminogruppen), Ammonium-Verbindungen, metallorganische Verbindungen und/oder Metallsalze, vorzugsweise von Zinn, Eisen, Bismut, Kalium und/oder Zink. Insbesondere können als Katalysatoren Gemische mehrerer solcher Verbindungen eingesetzt werden.

Schaumstabilisatoren (d) und deren Einsatz bei der Herstellung von PU-Schäumen sind dem Fachmann bekannt. Als Schaumstabilisatoren können insbesondere oberflächenaktive Verbindungen (Tenside) eingesetzt werden. Der Einsatz von Schaumstabilisatoren ist optional. Bevorzugt werden Schaumstabilisatoren eingesetzt. Sie können dazu dienen, die gewünschte Zellstruktur und den Verschäumungsprozess zu optimieren. Es können im Rahmen dieser Erfindung insbesondere alle Si-haltigen Verbindungen eingesetzt werden, die die Schaumherstellung unterstützen (Stabilisierung, Zellregulierung, Zellöffnung, etc.). Diese Verbindungen sind aus dem Stand der Technik hinreichend bekannt. Besonders bevorzugt kann mindestens ein Schaumstabilisator auf Basis eines Polyethersiloxans eingesetzt werden. Entsprechende, im Sinne dieser Erfindung einsetzbare Siloxanstrukturen werden z.B. in den folgenden Patentschriften beschrieben, wobei die Verwendung allerdings nur in klassischen PU-Schäumen (z.B. als Formschaum, Matratze, Isolationsmaterial, Bauschaum, etc.) beschrieben ist: CN 103665385, CN 103657518, CN 103055759, CN 103044687, US 2008/0125503, US 2015/0057384, EP 1520870 A1, EP 1211279, EP 0867464, EP 0867465, EP 0275563. Neben oberflächenaktiven Si-haltigen Verbindungen, können auch Si-freie Tenside eingesetzt werden. So wird beispielsweise in EP2295485 A1 die Verwendung von Lecithin und in US 3746663 die Verwendung von Vinylpyrrolidon-basierten Strukturen als Schaumstabilisator zur Herstellung von PU-Hartschaum beschrieben. Weitere Si-freie Schaumstabilisatoren sind beispielsweise in EP 2511328 B1, DE 1020011007479 A1, DE 3724716 C1, EP 0734404, EP 1985642, DE 2244350 und US 5236961 beschrieben.

Die Verwendung von Treibmitteln e) ist optional, je nachdem welches Verschäumungsverfahren verwendet wird. Es kann mit chemischen und physikalischen Treibmitteln gearbeitet werden.

Treibmittel und deren Einsatz bei der Herstellung von PU-Schäumen sind dem Fachmann bekannt. Die Verwendung von einem oder einer Kombination aus mehreren Treibmitteln (e) ist grundsätzlich abhängig von der Art des verwendeten Verschäumungsverfahrens, der Art des Systems und der Anwendung des erhaltenen PU-Schaums. Es können chemische und/oder physikalische Treibmittel als auch eine Kombination aus beiden verwendet werden. Je nach Menge des verwendeten Treibmittels wird ein Schaum mit hoher oder niedriger Dichte hergestellt. So können Schäume mit Dichten von 5 bis 900 kg/m3, bevorzugt 5 bis 350 kg/m3, besonders bevorzugt 8 bis 200 kg/m³, insbesondere 8 bis 150 kg/m³ hergestellt werden.

Als physikalische Treibmittel können alle entsprechenden Verbindungen mit passenden Siedepunkten sowie deren Mischungen, wie z.B. Kohlenwasserstoffe mit 3, 4 oder 5 Kohlenstoffatomen, bevorzugt cyclo-, iso-, n-Pentan, Fluorkohlenwasserstoffe (HFC), bevorzugt HFC 245fa, HFC 134a oder HFC 365mfc, Fluorchlorkohlenwasserstoffe (HCFC), bevorzugt HCFC 141b, Hydrofluoroolefine (HFO) oder Hydrohaloolefine, bevorzugt 1234ze, 1234yf, 1224yd, 1233zd(E) oder 1336mzz, Ester, bevorzugt Methylformiat, Ketone, bevorzugt Aceton, Ether bevorzugt, Dimethoxymethan, oder Chlorkohlenwasserstoffe, bevorzugt Dichlormethan oder 1,2-Dichlorethan eingesetzt werden.

Als chemische Treibmittel können alle Verbindungen eingesetzt werden, die mit NCO-Gruppen unter Freisetzung von Gasen reagieren, wie z.B. Wasser oder Ameisensäure oder durch den Temperaturanstieg während der Reaktion Gase freisetzen wie z.B. Natriumhydrogencarbonat.

Es entspricht einer besonders bevorzugten Ausführungsform, wenn die erfindungsgemäße Zusammensetzung als Treibmittel Wasser in Kombination mit Kohlenwasserstoffen mit 5 Kohlenstoffatomen, HFO, Hydrohaloolefine oder HFC oder Mischungen davon enthält.

Geeignete Wasser-Gehalte im Sinne dieser Erfindung hängen davon ab, ob zusätzlich zum Wasser noch ein oder mehrere Treibmittel eingesetzt werden oder nicht. Bei rein Wasser getriebenen Schäumen liegen bevorzugte Werte typischerweise bei 1 bis 20 pphp, werden zusätzlich andere Treibmittel eingesetzt, verringert sich die bevorzugte Einsatzmenge auf üblicherweise 0,1 bis 5 pphp.

Als Additive (f) können alle nach dem Stand der Technik bekannten Substanzen verwendet werden, die bei der Herstellung von Polyurethanen, insbesondere von Polyurethan-Schaumstoffen, Verwendung finden, wie zum Beispiel Vernetzer und Kettenverlängerer, Stabilisatoren gegen oxidativen Abbau (so genannte Antioxidantien), Flammschutzmittel, Tenside, Biozide, zellverfeinernde Additive, Zellöffner, feste Füllstoffe, Antistatik-Additive, Nukleierungsmittel, Verdicker, Farbstoffe, Pigmente, Farbpasten, Duftstoffe, Emulgatoren, usw.

Als Flammschutzmittel kann die erfindungsgemäße Zusammensetzung alle bekannten und zur Herstellung von PU-Schäumen geeigneten Flammschutzmittel wie z.B. halogenhaltige oder halogenfreie organische phosphorhaltige Verbindungen, wie z.B. Triethylphosphat (TEP), Tris(1-chlor-2-propyl)phosphat (TCPP, auch als Tris(2-chlorisopropyl)phosphat bezeichnet), Tris(2-chlorethyl)phosphat (TCEP), Dimethylmethanphosphonat (DMMP), Dimethylpropanphosphonat (DMPP), Ammoniumpolyphosphat oder roter Phosphor, stickstoffhaltige Verbindungen wie z.B. Melamin, Melamincyanurat oder Melaminpolyphosphat oder halogenierte Verbindungen, wie z.B. chlorierte und/oder bromierte Polyether- und/oder Polyesterpolyole enthalten. Es können auch Mischungen von verschiedenen Flammschutzmitteln eingesetzt werden. Vorzugsweise handelt es sich bei den Flammschutzmitteln um flüssige Flammschutzmittel.

Ein weiterer Gegenstand der Erfindung ist ein Verfahren zur Herstellung von Polyurethanschaum, durch Umsetzung mindestens einer Polyol-Komponente mit mindestens einer Polyisocyanat-Komponente unter Einsatz der erfindungsgemäßen Zusammensetzung.

Ein weiterer Gegenstand der Erfindung ist also ein Verfahren zur Herstellung von Polyurethanschaum, durch Umsetzung einer oder mehrerer Polyol-Komponenten mit mindestens einer oder mehrerer Polyisocyanat-Komponenten unter Einsatz der erfindungsgemäßen Zusammensetzung.

Das erfindungsgemäße Verfahren zur Herstellung von PU-Schäumen kann nach allen bekannten Methoden durchgeführt werden, z.B. im Handmischverfahren oder bevorzugt mit Hilfe von Verschäumungsmaschinen. Wird das Verfahren mittels Verschäumungsmaschinen durchgeführt, können Hochdruck- oder Niederdruckmaschinen verwendet werden. Das erfindungsgemäße Verfahren kann sowohl diskontinuierlich als auch kontinuierlich durchgeführt werden und es können z.B. 1K, 1.5K oder 2K Systeme wie in EP 3717538 A1, US 7776934 B2, EP 1400547 B1 oder EP 2780384 B2 beschrieben, verwendet werden.

Eine bevorzugte Polyurethan- bzw. Polyisocyanurat-Schaumformulierung im Sinne dieser Erfindung ergibt ein Raumgewicht von 5 bis 900 kg/m3 und hat die in Tabelle 1 genannte Zusammensetzung.

**Tabelle 1: Zusammensetzung einer bevorzugten Polyurethan- bzw. Polyisocyanurat-Hartschaumformulierung**

| Komponente | Gewichtsanteil |
|---|---|
| Polyol (erfindungsgemäße und weitere...) | 0,1 bis 100 |
| Amin-Katalysator | 0 bis 5 |
| Metall-Katalysator | 0 bis 10 |
| Surfactants | 0 bis 8 |
| Wasser | 0,01 bis 20 |
| Treibmittel | 0 bis 40 |
| Weitere Additive (Flammschutzmittel etc.) | 0 bis 90 |
| Isocyanat-Index: 10 bis 1000 | |

Für weitere bevorzugte Ausführungsformen und Ausgestaltungen des erfindungsgemäßen Verfahrens sei außerdem auf die zuvor bereits im Zusammenhang mit der erfindungsgemäßen Zusammensetzung gemachten Ausführungen verwiesen. Vorzugsweise gelten diese Ausführungen.

Ein weiterer Gegenstand der Erfindung ist ein Polyurethanschaumstoff, erhältlich durch das genannte Verfahren.

Gemäß einer bevorzugten Ausführungsform der Erfindung weist der Polyurethanschaum ein Raumgewicht von 5 bis 900 kg/m³, bevorzugt 8 bis 800 kg/m³, noch bevorzugter 10 bis 600 kg/m³, insbesondere 30 bis 150 kg/m³ auf.

Vorzugsweise handelt es sich beim erfindungsgemäßen Polyurethanschaum (PU-Schaum) um einen Polyurethan-Hartschaum (PU-Hartschaum).

"Polyurethan-Hartschaum" bzw. "PU-Hartschaum" ist ein feststehender technischer Begriff. Der bekannte und prinzipielle Unterschied zwischen Weichschaum und Hartschaum ist, dass ein Weichschaum ein elastisches Verhalten zeigt und damit die Verformung reversibel ist. Der Hartschaum wird demgegenüber dauerhaft verformt. Im Rahmen der vorliegenden Erfindung wird unter Polyurethan-Hartschaumstoff insbesondere ein Schaumstoff gemäß DIN 7726 verstanden, der eine Druckfestigkeit nach DIN 53 421 / DIN EN ISO 604 von vorteilhafterweise ≥ 20 kPa, vorzugsweise ≥ 80 kPa, bevorzugt ≥ 100 kPa, weiter bevorzugt ≥ 150 kPa, besonders bevorzugt ≥ 180 kPa aufweist. Weiterhin verfügt der Polyurethan-Hartschaumstoff nach DIN ISO 4590 vorteilhafterweise über eine Geschlossenzelligkeit von größer 50%, vorzugsweise größer 80% und besonders bevorzugt größer 90%. Weiteres zu Polyurethan-Hartschaumstoffen findet man auch im "Kunststoffhandbuch, Band 7, Polyurethane", Carl Hanser Verlag, 3. Auflage 1993, Kapitel 6 beschrieben.

Die PU-Schäume, insbesondere PU-Hartschäume, können als oder zur Herstellung von Isoliermaterialien, vorzugsweise Dämmplatten, Kühlschränken, Isolierschäumen, Dachhimmeln, Verpackungsschäumen oder Sprühschäumen verwendet werden.

Ein weiterer Gegenstand der vorliegenden Erfindung ist daher die Verwendung von erfindungsgemäßen Polyurethan- oder Polyisocyanurat-Schaumstoffen als Dämmplatten und/oder Isolationsmittel, vorzugsweise für Kühlapparaturen. Die Kühlapparaturen weisen dabei vorzugsweise als Isoliermaterial den erfindungsgemäßen Polyurethan- oder Polyisocyanurat-Schaumstoff auf.

Insbesondere in der Kühlhaus-, Kühlgeräte- und Hausgeräteindustrie; z.B. zur Herstellung von Dämmplatten für Dächer und Wände, als Isoliermaterial in Containern und Lagerhäusern für tiefgekühlte Ware sowie für Kühl- und Gefriergeräte, können die erfindungsgemäßen PU-Schäume mit Vorteil eingesetzt werden.

Weitere bevorzugte Anwendungsfelder liegen im Fahrzeugbau, insbesondere zur Herstellung von Fahrzeughimmel, Karosserieteilen, Innenverkleidungen, Kühlfahrzeugen, Großcontainern, Transportpaletten, Verpackungslaminaten, in der Möbelindustrie, z.B. für Möbelteile, Türen, Verkleidungen, in Elektronikanwendungen.

Erfindungsgemäße Kühlapparaturen weisen als Isoliermaterial einen erfindungsgemäßen PU-Schaum (Polyurethan- oder Polyisocyanurat-Schaumstoff) auf.

Ein weiterer bevorzugter Gegenstand der Erfindung liegt in der Verwendung des PU-Hartschaums als Isolationsmaterial in der Kältetechnik, in Kühlmöbeln, im Bau-, Automobil-, Schiffbau- und/oder Elektronikbereich, als Dämmplatten, als Sprühschaum, als Einkomponentenschaum.

Die Isoliereigenschaften von Polyurethanschäumen werden durch die Verwendung von Verbindungen der Formel (I) verbessert.

Ein weiterer Gegenstand ist daher auch die Verwendung von Verbindungen der Formel (I) oder ihren Mischungen zur Verbesserung der Isoliereigenschaften von Polyurethanschaumstoffen, insbesondere unter Einsatz einer erfindungsgemäßen Zusammensetzung, wie oben beschrieben.

Die erfindungsgemäßen Gegenstände werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Allgemeine Methoden:

### Polydispersität, massenmittlere molare Masse (Mw) und zahlenmittlere molare Masse (Mn):

Zur Bestimmung der Polydispersität (M_{w}/Mₙ), der massenmittleren molaren Masse (Mw) und der zahlenmittleren molaren Masse (Mₙ) wurden GPC-Messungen unter den folgenden Messbedingungen durchgeführt: Säulenkombination SDV 1000/10000 Å (Länge 65 cm), Temperatur 30 °C, THF als mobile Phase, Fließrate 1 ml/min, Probenkonzentration 10 g/l, RI-Detektor, Auswertung gegen Polypropylenglykol-Standard.

### Säurezahl:

Die Säurezahlbestimmung wurde nach einem Titrationsverfahren in Anlehnung an die DIN EN ISO 2114 durchgeführt.

### Hydroxylzahl (OH-Zahl):

Hydroxylzahlen wurden nach der Methode DGF C-V 17 a (53) der Deutschen Gesellschaft für Fettwissenschaft bestimmt. Dabei wurden die Proben mit Essigsäureanhydrid in Gegenwart von Pyridin acetyliert und der Verbrauch an Essigsäureanhydrid durch Titration mit 0,5 n Kalilauge in Ethanol gegen Phenolphthalein bestimmt.

### Viskosität:

Die Viskositäten wurden in Anlehnung an die DIN 53019 mit einem Rotationsviskosimeter der Marke Haake RV12 bei 25°C gemessen.

### Synthesebeispiele:

### Polyol Nr. 1: Isopropanolamid der Phthalsäure - Propoxyliert mit 6 PO (erfindungsgemäß)

In einem mit Vakuumpumpe, Destillatvorlage und Kühlfalle (gefüllt mit Aceton/Trockeneis) ausgestatteten 15-Liter-Reaktor wurden 1143 g Dimethylphthalat, 884 g Isopropanolamin und 65,3 g Natriummethanolat-Lösung (30 Gew.-% in Methanol) bei Raumtemperatur vorgelegt. Der Reaktorinhalt wurde durch mehrmaliges Aufdrücken von Stickstoff und anschließendes Entspannen inertisiert. Unter Rühren und bei ansteigender Temperatur bis 115 °C wurde Methanol innerhalb von 2 h unter Normaldruck abdestilliert. Die Viskosität des Reaktionsgemischs stieg dabei kontinuierlich an. Der Druck wurde auf 20 mbar abgesenkt und das restliche Methanol bei 115 °C unter Vakuum destillativ entfernt. Es entstand eine bei 115 °C gut rührbare Schmelze des aromatischen Amids. Das Ventil zur Vakuumpumpe und Destillatvorlage wurde geschlossen. Innerhalb von 1 h wurden 2058 g Propylenoxid bei 115 °C und einem Reaktorinnendruck von max. 3,5 bar (abs.) unter Rühren und Kühlen zugefügt. Die Viskosität des Reaktionsgemischs nahm im Laufe der Propoxylierung langsam ab. Nach 75 min Nachreaktion bei 115 °C fiel der Reaktorinndruck nicht weiter ab und die Umsetzung war beendet. Unter Vakuum wurden flüchtige Verbindungen wie restliches Propylenoxid destillativ entfernt. Das alkalische Produkt wurde auf 90 °C abgekühlt und zur Neutralisation mit 36,2 g Milchsäure (90 Gew.-% gelöst in Wasser) versetzt. Nach einer Rührzeit von 30 min wurde das fertige Polyol über einen Filter abgelassen. Ausbeute: 3697 g eines klaren Polyols mit folgenden Eigenschaften: Säurezahl: 0,1 mg KOH/g, OH-Zahl: 315 mg KOH/g, Viskosität (25 °C): 1679 mPas, M_{w}: 242 g/mol, Mₙ: 193 g/mol, M_{w}/Mₙ: 1,25.

### Polyol Nr. 2: Diisopropanolamid der Phthalsäure - Propoxyliert mit 3 PO (erfindungsgemäß)

In einem mit Vakuumpumpe, Destillatvorlage und Kühlfalle (gefüllt mit Aceton/Trockeneis) ausgestatteten 5-Liter-Reaktor wurden 197,5 g Dimethylphthalat, 266,4 g Diisopropanolamin und 11,0 g Natriummethanolat-Lösung (30 Gew.-% in Methanol) bei Raumtemperatur vorgelegt. Der Reaktorinhalt wurde durch mehrmaliges Aufdrücken von Stickstoff und anschließendes Entspannen inertisiert. Unter Rühren und bei ansteigender Temperatur bis 115 °C wurde Methanol innerhalb von 1 h 45 min unter Normaldruck abdestilliert. Die Viskosität des Reaktionsgemischs stieg dabei kontinuierlich an. Der Druck wurde auf 20 mbar abgesenkt und das restliche Methanol bei 115 °C unter Vakuum destillativ entfernt. Es entstand eine bei 115 °C gut rührbare Schmelze des aromatischen Amids. Das Ventil zur Vakuumpumpe und Destillatvorlage wurde geschlossen. Innerhalb von 3 h 15 min wurden 175 g Propylenoxid bei 115 °C und einem Reaktorinnendruck von max. 3,6 bar (abs.) unter Rühren und Kühlen zugefügt. Die Viskosität des Reaktionsgemischs nahm im Laufe der Propoxylierung langsam ab. Nach 3 h Nachreaktion bei 115 °C fiel der Reaktorinndruck nicht weiter ab und die Umsetzung war beendet. Unter Vakuum wurden flüchtige Verbindungen wie restliches Propylenoxid destillativ entfernt. Das alkalische Produkt wurde auf 90 °C abgekühlt und zur Neutralisation mit 6,1 g Milchsäure (90 Gew.-% gelöst in Wasser) versetzt. Nach einer Rührzeit von 30 min wurde das fertige Polyol über einen Filter abgelassen. Ausbeute: 520 g eines klaren Polyols mit folgenden Eigenschaften: Säurezahl: 0,2 mg KOH/g, OH-Zahl: 458 mg KOH/g, Viskosität (25 °C): >100000 mPas (nicht genau messbar), M_{w}: 457 g/mol, Mₙ: 270 g/mol, M_{w}/Mₙ: 1,69.

### Polyol Nr. 3: Diisopropanolamid der Phthalsäure - Propoxyliert mit 4 PO (erfindungsgemäß)

In einem mit Vakuumpumpe, Destillatvorlage und Kühlfalle (gefüllt mit Aceton/Trockeneis) ausgestatteten 5-Liter-Reaktor wurden 197,5 g Dimethylphthalat, 266,4 g Diisopropanolamin und 11,0 g Natriummethanolat-Lösung (30 Gew.-% in Methanol) bei Raumtemperatur vorgelegt. Der Reaktorinhalt wurde durch mehrmaliges Aufdrücken von Stickstoff und anschließendes Entspannen inertisiert. Unter Rühren und bei ansteigender Temperatur bis 115 °C wurde Methanol innerhalb von 1 h 45 min unter Normaldruck abdestilliert. Die Viskosität des Reaktionsgemischs stieg dabei kontinuierlich an. Der Druck wurde auf 20 mbar abgesenkt und das restliche Methanol bei 115 °C unter Vakuum destillativ entfernt. Es entstand eine bei 115 °C gut rührbare Schmelze des aromatischen Amids. Das Ventil zur Vakuumpumpe und Destillatvorlage wurde geschlossen. Innerhalb von 3 h 30 min wurden 232 g Propylenoxid bei 115 °C und einem Reaktorinnendruck von max. 3,6 bar (abs.) unter Rühren und Kühlen zugefügt. Die Viskosität des Reaktionsgemischs nahm im Laufe der Propoxylierung langsam ab. Nach 3 h Nachreaktion bei 115 °C fiel der Reaktorinndruck nicht weiter ab und die Umsetzung war beendet. Unter Vakuum wurden flüchtige Verbindungen wie restliches Propylenoxid destillativ entfernt. Das alkalische Produkt wurde auf 90 °C abgekühlt und zur Neutralisation mit 6,1 g Milchsäure (90 Gew.-% gelöst in Wasser) versetzt. Nach einer Rührzeit von 30 min wurde das fertige Polyol über ein einen Filter abgelassen. Ausbeute: 565 g eines klaren Polyols mit folgenden Eigenschaften: Säurezahl: 0,1 mg KOH/g, OH-Zahl: 419 mg KOH/g, Viskosität (25 °C): ca. 100000 mPas (nicht genau messbar), M_{w}: 472 g/mol, Mₙ: 308 g/mol, M_{w}/Mₙ: 1,53.

### Polyol Nr. 4: Diethanolamid der Phthalsäure - Propoxyliert mit 4 PO (nicht erfindungsgemäß)

In einem mit Vakuumpumpe, Destillatvorlage und Kühlfalle (gefüllt mit Aceton/Trockeneis) ausgestatteten 5-Liter-Reaktor wurden 873,9 g Dimethylphthalat, 946,4 g Diethanolamin und 49,5 g Natriummethanolat-Lösung (30 Gew.-% in Methanol) bei Raumtemperatur vorgelegt. Der Reaktorinhalt wurde durch mehrmaliges Aufdrücken von Stickstoff und anschließendes Entspannen inertisiert. Unter Rühren und bei ansteigender Temperatur bis 115 °C wurde Methanol innerhalb von 2,5 h min unter Normaldruck abdestilliert. Die Viskosität des Reaktionsgemischs stieg dabei kontinuierlich an. Der Druck wurde auf 20 mbar abgesenkt und das restliche Methanol bei 115 °C unter Vakuum destillativ entfernt. Es entstand eine bei 115 °C gut rührbare Schmelze des aromatischen Amids. Das Ventil zur Vakuumpumpe und Destillatvorlage wurde geschlossen. Innerhalb von 1 h wurden 1046 g Propylenoxid bei 115 °C und einem Reaktorinnendruck von max. 2,9 bar (abs.) unter Rühren und Kühlen zugefügt. Die Viskosität des Reaktionsgemischs nahm im Laufe der Propoxylierung langsam ab. Nach 2 h Nachreaktion bei 115 °C fiel der Reaktorinndruck nicht weiter ab und die Umsetzung war beendet. Unter Vakuum wurden flüchtige Verbindungen wie restliches Propylenoxid destillativ entfernt. Das alkalische Produkt wurde auf 90 °C abgekühlt und zur Neutralisation mit 27,5 g Milchsäure (90 Gew.-% gelöst in Wasser) versetzt. Nach einer Rührzeit von 30 min wurde das fertige Polyol über einen Filter abgelassen. Ausbeute: 2563 g eines klaren Polyols mit folgenden Eigenschaften: Säurezahl: 0,2 mg KOH/g, OH-Zahl: 426 mg KOH/g, Viskosität (25 °C): 55623 mPas, Mw: 515 g/mol, Mₙ: 242 g/mol, Mw/Mn: 2,12.

### Kommerziell erhältliche Rohstoffe:

- Lupranol^{®} 3422:: Polyetherpolyol basierend auf Sorbitol und Propylenoxid von BASF
- Daltolac^{®} R 251:: Polyetherpolyol basierend auf Glycerin und Propylenoxid von Huntsman
- Daltolac^{®} R 471:: Polyetherpolyol von Huntsman
- POLYCAT^{®} 5:: Amin-basierender Katalysator von Evonik Operations GmbH
- POLYCAT^{®} 8:: Amin-basierender Katalysator von Evonik Operations GmbH
- POLYCAT^{®} 41:: Amin-basierender Katalysator von Evonik Operations GmbH
- Desmodur^{®} 44V20L:: Diphenylmethan-4,4'-diisocyanat (MDI) mit Isomeren und höherfunktionellen Homologen von Covestro
- TEGOSTAB^{®} B 8491:: Polyether-Polydimethylsiloxan-Copolymer (Tensid) von Evonik Operations GmbH

### Herstellung von Polvol-Mischunqen

Um das Löslichkeitsverhalten der Polyole zu vergleichen, wurden die in Tabelle 1 beschriebenen Polyol-Mischungen hergestellt. Dabei wurde die Menge von Cyclopentan variiert und untersucht ab welcher Menge von Cyclopentan die Polyol-Mischungen nicht mehr klar sind sondern eintrüben. Diese Menge wird im Folgenden als Maximal-Menge bezeichnet.

**Tabelle 1: Polyol-Mischungen (Mengenangaben in Gewichtsteilen)**

| Polyol-Mischung | Nr. 1 | Nr. 2 | Nr. 3 | Nr. 4 |
|---|---|---|---|---|
| Lupranol^{®} 3422 | | | | |
| Daltolac^{®} R 251 | 80 | 80 | 80 | 80 |
| Polyol Nr. 1 (erfindungsgemäß) | 20 | | | |
| Polyol Nr. 2 (erfindungsgemäß) | | 20 | | |
| Polyol Nr. 3 (erfindungsgemäß) | | | 20 | |
| Polyol Nr. 4 (nicht erfindungsgemäß) | | | | 20 |
| POLYCAT^{®} 5 | 2,3 | 2,3 | 2,3 | 2,3 |
| POLYCAT^{®} 8 | 0,5 | 0,5 | 0,5 | 0,5 |
| POLYCAT^{®} 41 | 1,5 | 1,5 | 1,5 | 1,5 |
| Wasser | 2,5 | 2,5 | 2,5 | 2,5 |
| TEGOSTAB^{®} B 8491 | 2,5 | 2,5 | 2,5 | 2,5 |
| Cyclopentan (Maximal-Menge) | 17 | 10 | 11 | 8 |

Es ist zu erkennen, dass die erfindungsgemäßen Polyole Nr. 1, 2 und 3, welche lediglich Oxypropylen-Einheiten umfassen, im Gegensatz zum nicht erfindungsgemäßen Polyol Nr. 4, welches neben Oxypropylen-Einheiten zusätzlich noch Oxyethylen-Einheiten umfasst, auch bei einer höheren Menge an Cyclopentan noch zu klaren Mischungen führen. Dies ist besonders vorteilhaft für die Bereitstellung von sogenannten Polyol-Systemen, also solchen Zusammensetzungen, die alle Komponenten, die für die Herstellung eines PU-Schaums notwendig sind, enthalten, bis auf die Polyisocyanat-Komponente. Solche Polyol-Systeme werden bevorzugt bei der Herstellung von Kühlschränken eingesetzt. Hier ist es wichtig, dass das Polyol-System lagerstabil ist und es zu keiner Phasentrennung (z.B. eine mit bloßem Auge sichtbare Ausbildung von Schichten, ähnlich dem "Aufrahmen" bei Emulsionen) kommt, was bei trüben Emulsionen immer ein Risiko darstellt.

### Herstellung von PU-Schäumen

Die Durchführung der Verschäumungen erfolgte mit einer Verschäumungsmaschine von KraussMaffei, Modell RIM-Star MiniDos MK 12 ausgestattet mit einem Mischkopf Modell 8ULP-2KV-G-80. Hierbei wurden alle Komponenten gemäß Tabelle 3 mit Ausnahme des Polyisocyanates im Polyol-Tank vermischt und das Polyisocyannat im Isocyanat-Tank vorgelegt. Zur Verschäumung wurden die Komponenten mit den fogenden Drücken ausgetragen: Polyol-Komponente mit 130 bar, Polyoisocyanat mit 140 bar.

Die Reaktionsmischung wurde in eine auf 45°C thermostatisierte Bosch-Lanzen-Form mit den Abmessungen 200 cm × 20 cm × 5 cm eingetragen. Die Einsatzmenge an Schaumformulierung war dabei so bemessen, dass die Menge 15% über der Mindestbefüllung der Form lag. Die Schäume wurden nach 10 Minuten entformt und anschließend für 24 Stunden bei Raumtemperatur gelagert.

An den gehobelten Oberflächen der Schäume (Ober- und Unterseite) wurde der Grad der Störungen anhand einer Skala von 1 bis 10 visuell beurteilt, wobei 10 einen ungestörten Schaum und 1 einen extrem stark gestörten Schaum repräsentiert.

Die Wärmeleitzahl (*λ* -Wert in mW/(m·K)) wurde an 2,5 cm dicken Scheiben mit einem Gerät vom Typ Hesto Lambda Control, Modell HLC X206 nach 24 Stunden gemessen bei einer mittleren Temperatur von 10°C entsprechend den Vorgaben der Norm EN 12667:2001-05.

Zur Untersuchung der Nachexpansion wurde die Reaktionsmischung in eine auf 45°C temperierte Form mit den Abmessungen 50 cm (Tiefe) × 50 cm (Breite) × 10 cm (Höhe) eingetragen. Die Einsatzmenge an Schaumformulierung war dabei so bemessen, dass die Menge 15% über der Mindestbefüllung der Form lag.

Die Schäume wurden dann jeweils nach 4, 5 und 6 Minuten aus der Form entnommen und ihre Höhen direkt nach der Entformung und 24 Stunden nach der Entformung bestimmt. Hierbei ist eine möglichst geringe Zunahme der Höhe gewünscht. Im Idealfall wäre der Schaum also 10 cm hoch (Höhe der Form) und hätte damit keine Nachexpansion. Die Nachexpansion wird im Folgenden als prozentuale Änderung der Schaumhöhe bezogen auf den Ausgangswert angegeben. Der Ausgangswert entspricht der Höhe der Form (10 cm). Eine Nachexpansion von 10% bedeutet also eine Schaumhöhe von 11 cm. Eine Nachexpansion von 0% bedeutet also eine Schaumhöhe von 10 cm, d.h. es fand keine Nachexpansion statt.

In Tabelle 2 sind die verwendeten Schaumformulierungen zusammengefasst. Die Dosierung des Katalysators wurde bei den Schaumformulierungen so angepasst, dass alle Schaumformulierungen eine Gelzeit von 60 Sekunden hatten. Die Menge an Polyisocyanat (Desmodur^{®} 44V20L) wurde so gewählt, dass der Index der Formulierung 125 betrug.

**Tabelle 2: PU-Schaum-Formulierungen (Mengenangaben in Gewichtsteilen)**

| Formulierung | 1 | 2 | 3 (Vergleich) |
|---|---|---|---|
| Lupranol^{®} 3422 | 20 | 20 | 34 |
| Daltolac^{®} R 251 | 40 | 40 | 66 |
| Polyol Nr. 1 | 40 | | |
| Polyol Nr. 2 | | 40 | |
| POLYCAT^{®} 5 | 1,4 | 1,23 | 1,52 |
| POLYCAT^{®} 8 | 0,3 | 0,26 | 0,33 |
| POLYCAT^{®} 41 | 0,7 | 0,61 | 0,76 |
| Wasser | 2,5 | 2,5 | 2,5 |
| TEGOSTAB^{®} B 8491 | 2,5 | 2,5 | 2,5 |
| Cyclopentan | 15 | 15 | 15 |
| Desmodur^{®} 44V20L (Index 125) | 153 | 168 | 152 |

In den Tabellen 3 und 4 sind die Ergebnisse der Verschäumungsversuche der PU-Schaum-Formulierungen aus Tabelle 2 zusammengefasst.

**Tabelle 3: Zusammenfassung der Verschäumungsversuche in der Bosch-Lanze mit 15% Überfüllung (λ-Wert und Grad der Störung).**

| Schaum | Form.-Nr. | *λ* -Wert (mW/(m·K)) | Grad der Störungen auf der Oberseite | Grad der Störungen auf der Unterseite |
|---|---|---|---|---|
| 1 | 1 | 19,7 | 6 | 6 |
| 2 | 2 | 19,5 | 6 | 6 |
| 3 (Vergleich) | 3 (Vergleich) | 20,1 | 6 | 6 |

Aus den Versuchen ist klar ersichtlich, dass die erfindungsgemäßen Polyole zu verbesserten Isolationseigenschaften führen, und zwar ohne Beeinträchtigung der Qualität des Schaumes.

**Tabelle 4: Nachexpansion**

| Schaum Beispiel | Form.-Nr. | 4 min frisch | 4 min 24 h | 5 min frisch | 5 min 24 h | 6 min frisch | 6 min 24 h |
|---|---|---|---|---|---|---|---|
| 4 | 1 | 12,4 % | 8,4 % | 11,0 % | 8,1 % | 9,9 % | 6,9 % |
| 5 | 2 | 9,9 % | 7,3 % | 7,7 % | 5,1 % | 6,4 % | 3,8 % |
| 6 (Vergleich) | 3 (Vergleich) | 13,8 % | 10,5 % | 12,8 % | 9,2 % | 11,0 % | 8,4 % |

Aus den Versuchen ist klar ersichtlich, dass die erfindungsgemäßen Polyole zu einer Verringerung der Nachexpansion führen.

## Patentansprüche

1. Verbindung der Formel (I), 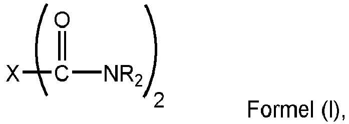 wobei
X ein Phenylenrest ist,
R jeweils unabhängig voneinander ausgewählt ist aus Resten der Formel (II), -[A-O]ₙ-H Formel (II),
A ein Propylenrest ist, und
n eine ganze Zahl ≥0 ist,
mit der Maßgabe, dass die Verbindung der Formel (I) mindestens 2 Einheiten A aufweist.

2. Verbindung der Formel (I) gemäß Anspruch 1, **dadurch gekennzeichnet, dass** X ein ortho-Phenylenrest ist.

3. Verbindung der Formel (I) gemäß Anspruch 1 oder 2, **dadurch gekennzeichnet, dass** für mindestens zwei Reste R gilt, dass n ungleich 0 ist.

4. Mischung umfassend oder bestehend aus Verbindungen der Formel (I) gemäß einem der Ansprüche 1 bis 3, **dadurch gekennzeichnet, dass** die mittlere Anzahl der Einheiten A pro Molekül der Verbindung der Formel (I) von 2 bis 14, vorzugsweise von 4 bis 12, insbesondere von 6 bis 10 beträgt.

5. Verfahren zur Herstellung von einer oder mehreren Verbindungen der Formel (I) gemäß einem der Ansprüche 1 bis 4, umfassend die Schritte:
a) Umsetzung mindestens einer aromatischen Verbindung ausgewählt aus der Gruppe bestehend aus Benzoldicarbonsäure, Benzoldicarbonsäureester und Benzoldicarbonsäureanhydrid mit mindestens einem Aminoalkohol ausgewählt aus der Gruppe bestehend aus Isopropanolamin und Diisopropanolamin, optional in Gegenwart eines alkalischen Katalysators, zu mindestens einem aromatischen Amid;
b) Umsetzung des mindestens einen aromatischen Amids mit Propylenoxid zu mindestens einer Verbindung der Formel (I).

6. Verfahren gemäß Anspruch 5, **dadurch gekennzeichnet, dass** in Schritt a) als aromatische Verbindung Dimethylphthalat eingesetzt wird.

7. Verfahren gemäß Anspruch 4 oder 5, **dadurch gekennzeichnet, dass** in Schritt a) und/oder in Schritt b) als alkalischer Katalysator wahlweise Natriummethanolat, Kaliummethanolat, NaOH oder KOH, bevorzugt Natriummethanolat oder Kaliummethanolat, besonders bevorzugt Natriummethanolat eingesetzt wird.

8. Verfahren gemäß einem der Ansprüche 5 bis 7, **dadurch gekennzeichnet, dass** in Schritt b) das Stoffmengenverhältnis von Propylenoxid zur Gesamtstoffmenge aller aromatischen Amide von 1 bis 10, vorzugsweise von 2 bis 8, insbesondere von 3 bis 6 beträgt.

9. Zusammensetzung zur Herstellung von Polyurethanschaum, umfassend mindestens eine Polyisocyanat-Komponente, mindestens eine Polyol-Komponente, optional mindestens einen Katalysator, der die Ausbildung einer Urethan- oder Isocyanurat-Bindung katalysiert, und optional mindestens ein Treibmittel, **dadurch gekennzeichnet, dass** mindestens eine Polyol-Komponente ausgewählt ist aus Verbindungen der Formel (I) gemäß einem der Ansprüche 1 bis 3 oder aus Mischungen gemäß Anspruch 4.

10. Zusammensetzung gemäß Anspruch 9, **dadurch gekennzeichnet, dass** der Massenanteil aller Verbindungen der Formel (I) bezogen auf die Gesamtmasse der Zusammensetzung von 3 % bis 40 %, vorzugsweise von 5 % bis 30 %, insbesondere von 10 % bis 20 % beträgt.

11. Zusammensetzung gemäß Anspruch 9 oder 10, **dadurch gekennzeichnet, dass** der Massenanteil aller Verbindungen der Formel (I) bezogen auf die Gesamtmasse aller Polyol-Komponenten von 5 % bis 65 %, vorzugsweise von 10 % bis 50%, insbesondere von 15 % bis 45 % beträgt.

12. Verfahren zur Herstellung von Polyurethanschaum, durch Umsetzung mindestens einer Polyol-Komponente mit mindestens einer Polyisocyanat-Komponente unter Einsatz einer Zusammensetzung gemäß einem der Ansprüche 9 bis 11.

13. Polyurethanschaumstoff, erhältlich durch das Verfahren gemäß Anspruch 12.

14. Verwendung des Polyurethanschaumstoffs gemäß Anspruch 13 als Dämmplatten und/oder Isolationsmittel, vorzugsweise für Kühlapparaturen.

15. Verwendung einer Verbindung der Formel (I) gemäß einem der Ansprüche 1 bis 3 oder einer Mischung gemäß Anspruch 4 zur Verbesserung der Isoliereigenschaften von Polyurethanschaumstoffen, insbesondere unter Einsatz einer Zusammensetzung gemäß einem der Ansprüche 9 bis 11.
